(19) Europäisches Patentamt
European Patent Office
Office européen des brevets

(11) EP 2 940 095 A1

(12) **EUROPEAN PATENT APPLICATION**

(43) Date of publication:
**04.11.2015 Bulletin 2015/45**

(21) Application number: **15168141.8**

(22) Date of filing: **15.12.2011**

(51) Int Cl.:
*C09K 8/60* (2006.01) *C09K 8/88* (2006.01)
*A01N 25/00* (2006.01) *C11D 3/37* (2006.01)
*A01N 25/10* (2006.01) *A61Q 19/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2010 US 423710 P**
**06.04.2011 EP 11161261**

(62) Document number(s) of the earlier application(s) in accordance with Art. 76 EPC:
**11801695.5 / 2 652 075**

(71) Applicant: **Akzo Nobel Chemicals International B.V.**
**3811 MH Amersfoort (NL)**

(72) Inventors:
- **Yuan-Huffman, Qingwen Wendy**
  **Belle Mead, NJ New Jersey 08502 (US)**
- **Rodrigues, Klin Aloysius**
  **Signal Mountain, TN Tennessee 37377 (US)**
- **Zhou, Jian**
  **Ridgefield, CT Connecticut 06877 (US)**
- **Holt, Stuart Peter Robert**
  **Highland Park, IL Illinois 60035 (US)**
- **Band, Elliot Isaac**
  **Pleasantville, NY New York 10570 (US)**
- **Vona Jr., Samuel Anthony**
  **Highland, NY New York 12528 (US)**

(74) Representative: **Akzo Nobel IP Department**
**Velperweg 76**
**6824 BM Arnhem (NL)**

Remarks:
This application was filed on 19-05-2015 as a divisional application to the application mentioned under INID code 62.

# (54) PERSONAL CARE COMPOSITIONS INCLUDING AQUEOUS COMPOSITIONS OF VISCOELASTIC SURFACTANTS AND HYDROPHOBICALLY MODIFIED POLYMERS

(57) Personal care compositions, methods for making and uses thereof include an aqueous viscoelastic composition and a personal care active ingredient. The viscoelastic composition includes at least one surfactant and at least one hydrophobically-modified polymers with low molecular weights.

Figure 1

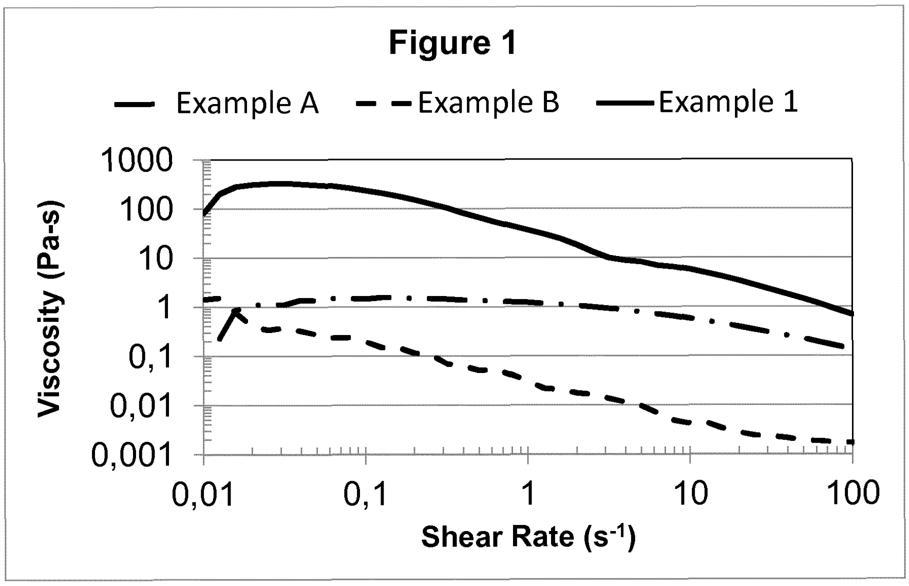


EP 2 940 095 A1

## Description

FIELD OF THE INVENTION

The present invention relates to personal care compositions comprising aqueous viscoelastic compositions comprising at least one viscoelastic surfactant, and at least one hydrophobically modified polymer.

BACKGROUND OF THE INVENTION

It is noted that WO 2003/056130 proposes an improvement on such existing systems and proposes to use a combination of viscoelastic surfactants and a hydrophobically modified polymer, wherein the concentration of the hydrophobically modified polymer is comprised between its overlap concentration c* and its entanglement concentration $c_e$. Although the viscoelastic fluids of WO 2003/056130 have certain commercial value, they contain high amounts of both surfactant and hydrophobically modified polymer in order to achieve aqueous compositions with the desired viscosity. Further, in the polymer, the hydrophobes are connected to the polymer backbone via a degradable group.

It is noted that also in US 4,432,881 liquids are used wherein a water-soluble polymer with hydrophobic groups is used. The polymers that are taught to be used have a weight averaged molecular weight of 200,000 to 5 million Dalton.

The high molecular weight polymers are difficult to dissolve and difficult to distribute homogeneously in aqueous formulations.

Personal cleansing products, such as shampoos, liquid hand soaps and body washes, not only must be able to clean the desired surfaces of the body, but they should also be aesthetically pleasing throughout all aspects of the consumer experience. For example, it is desirable that such cleansers should also provide pleasing experiences when dispensing the product, applying it, creating lather, cleansing, and rinsing the product.

It is desirable that liquid cleansing products to have a rich and luxurious aesthetic upon dispensing and application. Furthermore, consumers often equate a rich and luxurious lather to the cleansing efficacy of the product. Additionally, consumers have grown to expect additional benefits from their liquid cleansing products beyond cleansing. Such additional benefits include moisturization, conditioning, exfoliation, protection, and delivery of beneficial active ingredients to the hair and skin. Often times, these additional benefits are provided to the consumer by suspension or dispersion of active ingredients throughout the liquid cleansing product that are effectively deposited upon application.

Only low levels of surfactant are typically required to effectively clean the desired surfaces and that the level of surfactants in conventional liquid cleansing products vastly exceeds the required level. These high levels of surfactants are included in order to achieve several desirable attributes including: 1) rheological characteristics that are consistent with the rich and luxurious application aesthetic, 2) the rich and luxurious lather that consumers equate to product efficacy, and 3) rheological characteristics that enable shelf stable suspensions/dispersions of active ingredients.

In some cases, however, it is not possible to achieve the desired characteristics solely through increasing the quantity of surfactant. In these cases, the characteristics can be improved by addition of salt. Addition of salt to certain surfactant solutions induces a structural transition from spherical micelles to worm-like micelles. However, not all surfactant systems are sensitive to addition of salt in this way. Where salt is not effective, a variety of other types of components can be added to the surfactant systems to achieve the desired characteristics. Examples of such components include water soluble/dispersible thickeners that effectively thicken the system and chemicals that can crystallize to form complex three dimensional structures, such as PEG150 distearate,

The above thickeners tend to be high molecular weight species that effectively thicken aqueous systems. Some drawbacks to these thickeners are that they can be difficult to handle due to their high viscosity, poor dissolution speed, their relatively high level usage requirement and their negatively impact the clarity.

It is therefore desirable to provide chemicals that can more easily be incorporated into liquid cleansing products that enable rheological characteristics of surfactant systems, that enable suspension of benefit agents and that are consistent with the rich and luxurious aesthetic that consumers demand, while reducing the overall level of surfactant required to achieve these benefits.

SUMMARY OF THE INVENTION

High molecular weight polymers are generally difficult to dissolve and difficult to distribute homogeneously in aqueous formulations. Therefore, another solution is desired. More specifically, there is a need in the art for aqueous surfactant based systems with reduced amount of chemicals to obtain a certain viscosity or compositions with a higher viscosity when the same amount of chemicals are used, the amounts being based on the weight of the chemicals in the final composition, thereby further reducing the costs involved in the use of said fluid and/or expanding the applications wherein the compositions can be used. Also there is a need to be able to use polymers that are more easily dispersible in aqueous formulations.

It has been found that satisfactory aqueous viscoelastic compositions can be produced that do not suffer from the drawbacks of the conventional compositions of the art. More specifically, in an aspect, the invention provides a personal care composition comprising an aqueous viscoelastic composition comprising one or more viscoelastic surfactants in combination with one or more hydrophobically-modified polymers and a personal care active ingredient.

BRIEF DESCRIPTION OF THE DRAWING

The invention is best understood from the following detailed description when read in connection with the accompanying drawings. Included in the drawings are the following figures:

Figure 1 is chart illustrating the rheology profile determined by Steady Strain Sweep test for Example 1 and comparative examples A and B.

Figure 2 is chart illustrating the rheology profile determined by Steady Strain Sweep test for Example 2 and comparative examples C and D.

Figure 3 is a chart illustrating the rheology profile determined by Steady Strain Sweep test for comparative examples E and F.

Figure 4 is a chart illustrating the rheology profile determined by Steady Strain Sweep test for Example 5 and comparative examples H and I.

Figure 5 is a chart illustrating the rheology profile determined by Steady Strain Sweep test for Example 6 and comparative example J.

Figure 6 is a chart illustrating the results of steady state sweep measurements to determine zero shear viscosity.

Figure 7 is a chart illustrating the results of steady state sweep measurements to determine zero shear viscosity comparing fitted data to observed data.

Figure 8 is a chart showing the results of zero shear viscosity vs. % salt for 8 dilution experiments at various surfactant concentrations, four according to the present invention and four without the polymer-surfactant combination.

Figure 9 is a chart showing the results showing Elastic Modulus as a function of stress for two compositions prepared according to Examples 10 and 5, respectively.

DETAILED DESCRIPTION

The invention generally provides a personal care composition comprising an aqueous viscoelastic composition and a personal care active ingredient. The viscoelastic composition comprises one or more viscoelastic surfactants in combination with one or more hydrophobically-modified polymers.

In an embodiment, the viscoelastic surfactant is of the conventional type and can be selected from amine oxide surfactants including amidoamine oxide surfactants, amphoteric surfactants, zwitterionic surfactants, anionic surfactants, cationic surfactants and mixtures of two or more thereof.

The property of viscoelasticity in general is well known and reference is made to Hoffmann et al., "Influence of Ionic Surfactants on the Viscoelastic Properties of Zwitterionic Surfactant Solutions", Langmuir, 8, 2140-2146, (1992). A useful test method for viscoelasticity is to apply sinusoidal shear deformation to the composition and to measure the storage shear modulus (G') and the loss shear modulus (G") at a given temperature. If the elastic component (storage shear modulus G') is at least as large as the viscous component (loss shear modulus G"), that is $G' \geq G''$, at some point or over some range of points below a frequency of about 10 rad/sec, typically between about 0.001 to about 10 rad/sec, more typically between 0.1 to 10 rad/sec, at a given temperature and if $G' > 10^{-2}$ Pascal, preferably more than $10^{-1}$ Pascal, the fluid is considered viscoelastic at that temperature. The definition and rheological measurement of G' and G" are generally described in Barnes H. A. et al., "An Introduction to Rheology", pp.45-54, Elsevier, Amsterdam (1997).

The viscoelastic surfactant is of the conventional type. It is well known that viscoelastic surfactants provide viscoelasticity by forming a different type of micelle than the usual spherical micelles formed by most surfactants. Viscoelastic surfactants form elongated, often cylindrical, micelles which can be described as worm-like, thread-like, or rod-like micelles. Typically it is said that the shape and size of a micelle is a function of the molecular geometry of its surfactant molecules and solution conditions such as surfactant concentration, temperature, pH, and ionic strength. The formation

of long, cylindrical micelles creates useful rheological properties. Viscoelastic surfactant exhibits shear-thinning behavior, and remain stable despite repeated high shear applications. By comparison, a typical polymeric thickener will irreversibly degrade when subjected to high shear applications. In an embodiment, a viscoelastic surfactant is a surfactant that forms a visocelastic fluid in an aqueous media.

Amino Oxide and Amidoamine Oxide Viscoelastic Surfactant

In an embodiment, amine oxide surfactants contemplated for use in the present invention as viscoelastic surfactants include those of the following structural formula (I):

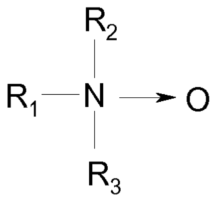

(I)

where $R_1$ is a hydrophobic moiety of alkyl, alkenyl, cycloalkyl, alkylarylalkyl, alkoxyalkyl, alkylaminoalkyl or alkylamidoalkyl. $R_1$ has from about 8 to about 30 carbon atoms and may be straight or branched chained and saturated or unsaturated. Examples of long chain alkyl groups include, but not limited to, octadecenyl (oleyl), octadecyl (stearyl), docosenoic (erucyl) and the derivatives of tallow, coco, soy and rapeseed oils.

$R_2$ and $R_3$ are, independently, hydrogen or aliphatic group (i.e. as opposed to aromatic) having from 1 to about 30 carbon atoms, preferably from about 1 to about 20 carbon atoms, more preferably from about 1 to about 10 carbon atoms, and most preferably from about 1 to about 6 carbon atoms. Representative aliphatic groups include alkyl, alkenyl, cycloalkyl, alkylaryl, hydroxyalkyl, carboxyalkyl and hydroxyalkyl-polyoxyalkylene. The aliphatic group can be straight or branched chained and saturated or unsaturated.

In an embodiment, amidoamine oxide surfactants contemplated for use as viscoelastic surfactants in the present invention include those of the following structural formula (II):

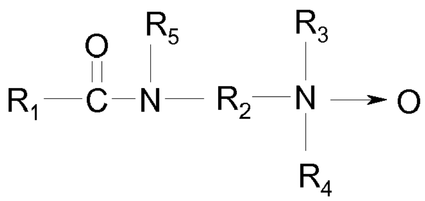

(II)

where $R_1$ is a straight or branched chained and saturated or unsaturated aliphatic group of from about 8 to about 30 carbon atoms, preferably from about 14 to about 21 carbon atoms. More preferably, R1 is a fatty aliphatic derived from natural fats and oils having an iodine value of from about 1 to about 140, preferably from about 30 to about 90, and more preferably from 40 to about 70. $R_1$ may be restricted to a single chain length or may be of mixed chain length such as those groups derived from natural fats and oils or petroleum stocks. Preferred are tallow alkyl, hardened tallow alkyl, rapeseed alkyl, hardened rapeseed alkyl, tall oil alkyl, hardened tall oil alkyl, coco alkyl, oleyl, or soya alkyl;

$R_2$ is a straight or branched-chained, substituted or unsubstituted, divalent alkylene group of from 2 to about 6 carbon atoms, preferably, of 2 to 4 carbon atoms and more preferably of 3 carbon atoms;

$R_3$ and $R_4$ are the same or different and are selected from alkyl or hydroxyalkyl groups of from 1 to about 4 carbon atoms and are preferably hydroxyethyl or methyl. Alternatively, $R_3$ and $R_4$ in the amidoamine oxide of formula (II) together with the nitrogen atom to which these groups are bonded form a heterocyclic ring of up to 6 members; and

$R_5$ is hydrogen or a $C_1$ - $C_4$ alkyl or hydroxyalkyl group.

Examples of amidoamine oxide contemplated by the present invention include, but are not limited to, those selected from the group consisting of tallow amidoalkylamine oxide, hardened tallow amidoalkylamine oxide, rapeseed amidoalkylamine oxide, hardened rapeseed amidoalkylamine oxide, tall oil amidoalkylamine oxide, hardened amidoalkylamine oxide, coco amidoalkylamine oxide, stearyl amidoalkylamine oxide, oleyl amidoalkylamine oxide, soya amidoalkylamine oxide, and mixtures thereof. Preferred specific examples of the amidoamine oxides of the present invention include, but are not limited to, the following: tallow amidopropyl dimethylamine oxide, hydrogenated tallow amidopropyl dimethylamine oxide, soya amidopropyl dimethylamine oxide, oleyl amidopropyl dimethylamine oxide, erucyl amidopropyl dimethylamine oxide, rapeseed amidopropyl dimethylamine oxide, hydrogenated rapeseed amidopropyl

dimethylamine oxide, tall oil amidopropyl dimethylamine oxide, hydrogenated tall oil amidopropyl dimethylamine oxide, C14 - C22 saturated or unsaturated fatty acid amidopropyl dimethylamine oxides, and mixtures thereof.

Cationic Viscoelastic Surfactant

A cationic surfactant has a positively charged moiety regardless of pH. In an embodiment, cationic surfactants contemplated for use as viscoelastic surfactants in the present invention include those selected from quaternary salts, certain amines and combinations thereof.

In an embodiment, the quaternary salts have the structural formula (III)

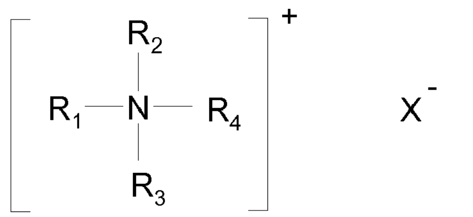

(III)

where $R_1$ is a hydrophobic moiety of alkyl, alkenyl, cycloalkyl, alkylarylalkyl, alkoxyalkyl, alkylaminoalkyl or alkylamidoalkyl. $R_1$ has from about 8 to about 30 carbon atoms and may be straight or branched chained and saturated or unsaturated. Examples of long chain alkyl groups include, but are not limited to, octadecenyl (oleyl), octadecyl (stearyl), docosenoic (erucyl) and the derivatives of tallow, coco, soy and rapeseed oils;

$R_2$, $R_3$ and $R_4$ are, independently, at least partially aliphatic groups having from 1 to about 30 carbon atoms, preferably from about 1 to about 20 carbon atoms, more preferably from about 1 to about 10 carbon atoms, and most preferably from about 1 to about 6 carbon atoms. Representative aliphatic groups include alkyl, alkenyl, alkylaryl, alkoxyalkyl, hydroxyalkyl, carboxyalkyl and hydroxyalkyl-polyoxyalkylene. The aliphatic group can be straight or branched chained and saturated or unsaturated, and;

$X^-$ is a suitable counter-anion. The counter-anion is typically an inorganic anion such as a sulfate such as $(CH_3)_2SO_4^-$, a nitrate, a perchlorate or a halide such as $Cl^-$, $Br^-$, or an aromatic organic anion such as salicylate, naphthalene sulfonate, p- and m-chlorobenzoates, 3,5-, 3,4-, and 2,4-dichlorobenzoates, t-butyl and ethyl phenate, 2,6- and 2,5-dichlorophenates, 2,4,5- trichlorophenate, 2,3,5,6-tetrachlorophenate, p-methyl phenate, m-chlorophenate, 3,5,6-trichloropicolinate, 4-amino- 3,5,6-, 2,4-dichlorophenoxyacetate.

The amines have the following structural formula (IV):

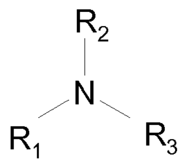

(IV)

where $R_1$, $R_2$ and $R_3$ have the meaning as defined above for the quaternary salt residues $R_1$, $R_2$ and $R_3$, respectively.

Zwitterionic Viscoelastic Surfactant

The zwitterionic surfactant has a permanently positively charged moiety in the molecule regardless of pH and a negatively charged moiety at alkaline pH. In an embodiment, selected zwitterionic surfactants that are useful as viscoelastic surfactants in the present invention have the following structural formula (V):

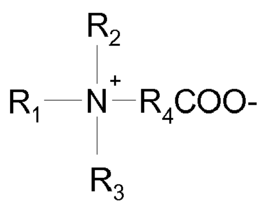

(V)

where $R_1$ is hydrophobic moiety of alkyl, alkenyl, cycloalkyl, alkylarylalkyl, alkoxyalkyl, alkylaminoalkyl or alkylamidoalkyl. $R_1$ has from about 8 to about 30 carbon atoms and may be straight or branched chained and saturated or unsaturated. Examples of long chain alkyl groups include, but are not limited to, octadecenyl (oleyl), octadecyl (stearyl), docosenoic (erucyl) and the derivatives of tallow, coco, soy and rapeseed oils;

$R_2$ and $R_3$ are, independently, at least partially aliphatic groups having from 1 to about 30 carbon atoms, preferably from about 1 to about 20 carbon atoms, more preferably from about 1 to about 10 carbon atoms, and most preferably from about 1 to about 6 carbon atoms. Representative aliphatic groups include alkyl, alkenyl, alkylaryl, alkoxyalkyl, hydroxyalkyl, carboxyalkyl and hydroxyalkyl-polyoxyalkylene. The aliphatic group can be straight or branched chained and saturated or unsaturated, and;

$R_4$ is a hydrocarbyl radical (e.g. alkylene) with a chain length of 1 to 4 carbon atoms. Preferred are methylene or ethylene groups.

When it is zwitterionic, the surfactant is associated with both negative and positive counter-ions. Anions are typically as defined above for $X^-$ for the cationic surfactant. In an embodiment any cation is suitably elected from $Na^+$, $K^+$, NH4+, and amine salts, such as $(CH_3)_2NH_2^+$.

Amphoteric Viscoelastic Surfactant

An amphoteric surfactant has both a positively charged moiety and a negatively charged moiety over a certain pH range (e.g. typically slightly acidic), only a negatively charged moiety over a certain pH range (e.g. typically slightly alkaline) and only a positively charged moiety at a different pH (e.g. typically moderately acidic).

In an embodiment, amphoteric surfactants suitable for use as viscoelastic surfactants in the present invention are represented by following the structural formula (VI):

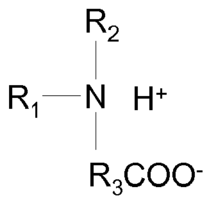

(VI)

where $R_1$ is a hydrophobic moiety of alkyl, alkenyl, cycloalkyl, alkylarylalkyl, alkoxyalkyl, alkylaminoalkyl or alkylamidoalkyl. $R_1$ has from about 8 to about 30 carbon atoms and may be straight or branched chained and saturated or unsaturated. Examples of long chain alkyl groups include, but are not limited to, octadecenyl (oleyl), octadecyl (stearyl), docosenoic (erucyl) and the derivatives of tallow, coco, soy and rapeseed oils;

$R_2$ has the meaning as defined above for the residue $R_2$ of the zwitterionic surfactant;

$R_3$ is a hydrocarbyl radical (e.g. alkylene) with a chain length of 1 to 4 carbon atoms. Preferred are methylene or ethylene groups.

Anionic Viscoelastic Surfactant

An anionic surfactant has a negatively charged moiety regardless of pH. In an embodiment, the anionic surfactants contemplated for use as viscoelastic surfactants in the present invention include those of the following structural formulas (VII), (VIII) and/or (VIIII).

R-Z (VII)

R-X-Y-Z (VIII)

R-Y-Z (VIIII)

where R is the hydrophobic moiety of alkyl, alkenyl, cycloalkyl, alkylarylalkyl, alkoxyalkyl, alkylaminoalkyl or alkylamidoalkyl. Preferably, R is a saturated or unsaturated, straight or branched alkyl chain of at least 8 carbon atoms,

and in another embodiment from 8 to 30 carbon atoms. Examples of long alkyl chain groups include, but not limited to, octadecenyl (oleyl), lauryl, octadecyl (stearyl), docosenoic (erucyl) and the derivatives of tallow, coco, soy and rapeseed oils;

Z is the negatively charged hydrophilic head of the surfactant. Z is suitably selected from the group consisting of carboxylate $COO^-$, sulfonate $SO_3^-$, sulfate $SO_4$ phosphonate, phosphate, and combinations thereofIn an embodiment, Z may be a carboxylate group $COO^-$ or a sulfonate group $SO_3^-$;

X is a stabilizing group. X is preferably a cleavable bond. Preferably, X is an ester, amid, reverse ester or reverse amide group;

Y is a space group, which separates the cleavable group X and the hydrophilic head of the surfactant. Y is preferably a linear, saturated or unsaturated hydrocarbon chain of 1, 2 or 3 carbon atoms or a branched, saturated or unsaturated hydrocarbon chain where the main chain is of 1, 2 or 3 carbon atoms, possibly incorporating an aromatic ring.

Optionally, the surfactant of the invention may be dimeric or oligomeric. In such case, the formula of the surfactant is $[R\text{-}Z]_n$ or $[R\text{-}X\text{-}Y\text{-}Z]_n$, where n is 2-10, preferably 2 or 3. An example of an oligomeric anionic surfactant is oligomerized oleic acid which generally leads to complex mixtures of dimeric and trimeric products. Commercially available oligomers, such as the Empol™ series of dimmers and trimers are suitable for use in accordance with the present invention.
When the surfactant is anionic, the counter-ion is typically $Na^+$, $K^+$, $NH_4^+$, or amine salt such as $(CH_3)_2NH_2^+$.
These mono-, di- or oligomeric carboxylates or sulfonates form viscoelastic aqueous compositions in the presence of salt.
Preferably the surfactants that are used are biodegrable, more preferably readily biodegradable, when testing using conventional tests such as OECD 306 A-F.
Surfactants suitable in the present invention include those known in the art for use in personal care compositions, and include nonionic, anionic, cationic, and amphoteric surfactants. Classes of surfactants useful in personal care compositions, such as personal cleansing compositions, of the present invention include the following: alcohols, alkanolamides, alkylaryl sulfonates, alkylaryl sulfonic acids, alkylbenzenes, amine acetates, amine oxides, amines, sulfonated amines and amides, betaines, block polymers, carboxylated alcohol or alkylphenol ethoxylates, diphenyl sulfonate derivatives, ethoxylated alcohols, ethoxylated alkylphenols, ethoxylated amines and/or amides, ethoxylated fatty acids, ethoxylated fatty esters and oils, fatty esters (other than glycol, glycerol, etc.), fluorocarbon-based surfactants, glycerol esters, glycol esters, heterocyclics, imidazolines and imidazoline derivatives, isethionates, lanolin-based derivatives, lecithin and lecithin derivatives, lignin and lignin derivatives, methyl esters, monoglycerides and derivatives, olefin sulfonates, phosphate esters, phosphorous organic derivatives, polymeric (polysaccharides, acrylic acid, acrylamide), propoxylated and ethoxylated fatty acids, propoxylated and ethoxylated fatty alcohols, propoxylated and ethoxylated alkyl phenols, protein-based surfactants, quaternary surfactants, sarcosine derivatives, silicone-based surfactants, soaps, sorbitan derivative, sucrose and glucose esters and derivatives, sulfates and sulfonates of oils and fatty acids, sulfates and sulfonates ethoxylated alkyl phenols, sulfates of alcohols, sulfates of ethoxylated alcohols, sulfates of fatty esters, sulfonates of benzene, cumene, toluene and xylene, sulfonates of condensed naphthalenes, sulfonates of dodecyl and tridecyl benzenes, sulfonates of naphthalene and alkyl naphthalene, sulfonates of petroleum, sulfosuccinamates, sulfosuccinates and derivatives.
Anionic surfactants suitable for use in the personal care compositions are the alkyl and alkyl ether sulfates. These materials have the respective formulae-- $ROSO_3M$ and $RO(C_2H_4O)_xSO_3M$ wherein R is alkyl or alkenyl of from about 8 to about 18 carbon atoms, x is an integer having a value of from 1 to 10, and M is a cation, such as ammonium, alkanolamine such as triethanolamine, monovalent metal, such as sodium or potassium, and polyvalent metal cation, such as magnesium or calcium. The cation M should be selected such that the anionic surfactant component is water soluble. Solubility of the surfactant depends upon the particular anionic surfactants and cations chosen.
In an embodiment, R has from about 8 to about 18 carbon atoms in both the alkyl and alkyl ether sulfates. In another embodiment, R has from about 10 to about 16 carbon atoms. In even another embodiment, R has from about 12 to about 14 carbon atoms. The alkyl ether sulfates are typically made as condensation products of ethylene oxide and monohydric alcohols having from about 8 to about 24 carbon atoms. The alcohols can be synthetic or they can be derived from fats (e.g., coconut oil, palm kernel oil, tallow). In an embodiment, lauryl alcohol and straight chain alcohols derived from coconut oil or palm kernel oil are preferred. Such alcohols are reacted with between about 0 and about 10, preferably from about 2 to about 5, more preferably about 3, molar proportions of ethylene oxide, and the resulting mixture of molecular species having, for example, an average of 3 moles of ethylene oxide per mole of alcohol, is sulfated and neutralized.
Specific non-limiting examples of alkyl ether sulfates which can be used in the personal care compositions of the present invention include, but are not limited to, sodium and ammonium salts of coconut alkyl triethylene glycol ether

sulfate, tallow alkyl triethylene glycol ether sulfate, and tallow alkyl hexaoxyethylene sulfate. In an embodiment, alkyl ether sulfates are those comprising a mixture of individual compounds, wherein the compounds in the mixture have an average alkyl chain length of from about 10 to about 16 carbon atoms and an average degree of ethoxylation of from about 1 to about 4 moles of ethylene oxide.

Other suitable anionic surfactants are the water-soluble salts of organic/sulfuric acid reaction products conforming to the formula-- [$R^1$--$SO_3$-M] where $R_1$ is a straight or branched chain, saturated, aliphatic hydrocarbon radical having from about 8 to about 24, preferably about 10 to about 18, carbon atoms; and M isa cation, such as ammonium, alkanolamine such as triethanolamine, monovalent metal, such as sodium or potassium, and polyvalent metal cation, such as magnesium or calcium. The cation M should be selected such that the anionic surfactant component is water soluble. Non limiting examples of such surfactants are the salts of an organic sulfuric acid reaction product of a hydrocarbon of the methane series, including iso-, neo-, and n-paraffins, having from about 8 to about 24 carbon atoms, preferably about 12 to about 18 carbon atoms and a sulfonating agent (e.g., $SO_3$, $H_2SO_4$) obtained by known sulfonation methods such as bleaching and hydrolysis. Preferred are alkali metal and ammonium sulfonated $C_{10}$ to $C_{18}$ n-paraffins.

Still other suitable anionic surfactants are the reaction products of fatty acids esterified with isethionic acid and neutralized with sodium hydroxide where, for example, the fatty acids are derived from coconut oil or palm kernel oil; sodium or potassium salts of fatty acid amides of methyl tauride in which the fatty acids, for example, are derived from coconut oil or palm kernel oil. Other similar anionic surfactants are described in U.S. Pat. Nos. 2,486,921, 2,486,922 and 2,396,278, each of which is incorporated by reference in its entirety herein.

Succinates are another type of anionic surfactant suitable for use in the personal care compositions of the present invention. Examples include disodium N-octadecylsulfosuccinnate; disodium lauryl sulfosuccinate; diammonium lauryl sulfosuccinate; tetrasodium N-(1,2-carboxyethyl)-N-octadecyl sulfosuccinate; diamyl ester of sodium sulfosuccinic acid; dihexyl ester of sodium sulfosuccinic acid; and dioctyl esters of sodium sulfosuccinic acid.

Other suitable anionic surfactants include olefin sulfonates having about 10 to about 24 carbon atoms. In this context the term "olefin sulfonates" refers to compounds produced by sulfonation of $\alpha$-olefins by means of uncomplexed sulfur trioxide, followed by neutralization of the acid reaction mixture in conditions such that any sulfones formed in the reaction are hydrolyzed to give the corresponding hydroxy alkane sulfonates. The sulfur trioxide can be liquid or gaseous, and is usually, but not necessarily, diluted by inert diluents (e.g., by liquid $SO_2$, chlorinated hydrocarbons, etc.) when used in the liquid form, or by air, nitrogen, gaseous $SO_2$, etc., when used in the gaseous form. Those $\alpha$-olefins from which the olefin sulfonates are derived are monoolefins having from about 10 to about 24 carbon atoms, and preferably from about 12 to about 16 carbon atoms. Preferably, they are straight chain olefins. In addition to true alkene sulfonates and a proportion of hydroxy alkane sulfonates, the olefin sulfonates can contain minor amounts of other materials, such as alkene disulfonates, depending upon reaction conditions, proportion of reactants, nature of the starting olefins and impurities in the olefin stock, and side reactions during the sulfonation process. Combinations of $\alpha$-olefins are also contemplated for use in the present invention. A non-limiting example of such an $\alpha$-olefin sulfonate mixture is described in U.S. Pat. No. 3,332,880, which is incorporated by reference in its entirety herein.

Another class of anionic surfactants suitable for use in the personal care compositions are the $\beta$-alkyloxy alkane sulfonates. Examples of suitable anionic surfactants for use in the personal care compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, arumonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, and combinations thereof.

Suitable amphoteric or zwitterionic detersive surfactants for use in the personal care composition herein include those which are known for use in hair care or other personal care cleansing composition, and which contain a group that is anionic at the pH of the personal care composition.

Amphoteric surfactants suitable for use in the personal care composition are well known in the art, and include those surfactants broadly described as derivatives of aliphatic secondary and tertiary amines in which the aliphatic radical can be straight or branched chain, wherein at least one of the aliphatic substituents contains from about 8 to about 18 carbon atoms and at least one contains an anionic water solubilizing group such as carboxy, sulfonate, sulfate, phosphate, or phosphonate.

Zwitterionic surfactants suitable for use in the personal care composition are well known in the art, and include those surfactants broadly described as derivatives of aliphatic quaternary ammonium, phosphonium, and sulfonium compounds in which the aliphatic radicals are straight or branched chain, and wherein at least one of the aliphatic substituents contains from about 8 to about 18 carbon atoms and at least one contains an anionic group such as carboxy, sulfonate, sulfate, phosphate or phosphonate. Zwitterionic surfactants such as betaines are preferred.

Non-limiting examples of other nonionic, anionic, zwitterionic, amphoteric or additional surfactants suitable for use in the personal care compositions are described in McCutcheon's Emulsifiers and Detergents 1989 Annual, MC Publishing, Glen Rock, N.J., as well as U.S. Pat. Nos. 3,929,678 and 2,528,378, each of which is incorporated by reference in its entirety herein.

Surfactants in the present invention can be used alone or in combination.

Hydrophobically modified polymers

The hydrophobically modified polymers can be anionic hydrophobically modified polymer or cationic hydrophobically modified polymer or non-ionic hydrophobically modified polymer or zwitterionic hydrophobically modified polymer.

The at least one hydrophobically modified polymer is formed from polymerization of ethylenically unsaturated monomers using polymerization conditions known to those skilled in the art.

The hydrophobically modified polymers have a number average molecular weight of from 1,000, preferably from 1,500, for more preferably from 2,500, to 100,000, more preferably to 90,000 and more preferably to 50,000, and even more preferably to 25,000 Da. In the context of this invention, the polymer molecular weights are as determined with size exclusion chromatography using HPLC grade water comprising 0.025M $NaH_2PO_4$, 0.025 M $Na_2HPO_4$, and 0.01 M of sodium azide which was filtered through a 0.2 $\mu$m filter as the eluent and four separation columns, G6000PWxl 7.8 mm x 30 cm, G4000PWxl 7.8 mm x 30 cm, G3000PWxl 7.8 mm x 30 cm, and TSKgel Guard PWxl 6.0 mm x 4 cm as the G2500 Guard column (all ex Tosoh Bioscience). Polyacrylic acid sodium salt standards (ex American Polymer Standards Corporation) were used for calibration. The polymers are prepared in water at a concentration of 0.1 %w/w. The weight average (Mw) and number average molecular weight (Mn) of the standards are:

| | | | | |
|---|---|---|---|---|
| 1. | Mw | 1,300 Dalton | Mn | 830 Dalton |
| 2. | Mw | 8,300 Dalton | Mn | 6200 Dalton |
| 3. | Mw | 83,400 Dalton Mn | 47,900 | Dalton |
| 4. | Mw | 495,000 Dalton | Mn | 311,300 Dalton |
| 5. | Mw | 1,700,000 Dalton | Mn | 1,100,000 Dalton |

Injection column was 450 $\mu$L for the standard and sample. Inject the standards and build a first-order or second-order calibration curve. Choose the curve with the best fit and within the range of the sample molecular weight. Run time was 60 minutes per injection for standard and sample.

Surprisingly, such low molecular weight polymers, that in themselves are typically not thickeners, were found to interact with the viscoelastic surfactants in a way that led to an increased viscosity which is much higher than the viscosity of solution of pure viscoelastic surfactant of the same concentration.

According to a non-binding theory, the hydrophobically-modified polymers of the invention, notably its pendant hydrophobic chains, interact in an improved way with the surfactant micelles. This interaction conceivably increases the worm- or rod-like micelle size, and/or cross-linked the micelles, so that a higher viscosity is achieved. As a result, an aqueous viscoelastic structure that satisfies the required rheology profile is obtained using less amount of chemicals than previously possible. At the same time the lower molecular weight polymers allow easier handling of the polymer itself and faster preparation of the compositions of the invention.

The hydrophobically-modified polymer has a backbone and, attached to said backbone, randomly or not, pendant hydrophobic chains. The polymer can be charged or non-charged, depending on the monomers used and the pH of the aqueous composition in which it is used. If too many hydrophobic groups are present, the water-solubility decreases. If it is too low, the interaction with the viscoelastic surfactant will become impaired. In an embodiment of the invention the amount of hydrophobic monomer-derived units is about 0.1 mole% or more. In another embodiment it is about 0.5 mole% or more and in a further embodiment it is about 1 mole% or more of all the monomer units comprised in the polymer.

To a level of at least 0.1 mole%, based on the amount of monomer units in the polymer, the hydrophobically modified polymer comprises monomeric units each covalently bonded to a pendant hydrocarbyl group having from 6 to 40 carbon atoms, said pendant hydrocarbyl group being connected to the backbone of said hydrophobically modified polymer via a non-ester containing linking group.

The pendant hydrocarbyl group has from 6, preferably from 8, more preferably from 11, for example from 14, to 40, preferably to 32, more preferred to 24 carbon atoms. The pendant hydrocarbyl group is typically a straight, branched or cyclic, saturated or unsaturated hydrocarbyl, such as a linear or branched alkyl, alkenyl, cycloalkyl, aryl, alkylaryl, or alkenylaryl group. Examples of pendant hydrocarbyl groups include behenyl, stearyl, lauryl, 2-etylhexyl, 2-propylheptyl, 2-butyloctyl, 2-hexyldecyl, 2-octyldodecyl, 2-decyltetradecyl, 2-dodecylhexadecyl, 2-tetradecyloctadecyl, or the alkyl group of oleyl, coco, soya, erucyl or tallow acids or alcohols or amines.

Suitably, the hydrophobically-modified polymer is synthesized by copolymerization of one or more hydrophilic,

one or more hydrophobic monomers, and optionally one or more other nonionic monomers. The monomers used can be copolymerized in any sequence. In an embodiment at least acrylic acid is used as a hydrophilic monomer. In another embodiment the hydrophobic monomer is selected from monomers that result in pendant hydrophobic chain, pendant of the backbone of said polymer, having at least 8 carbon atoms in said pendant chain. In an embodiment of the invention the amount of hydrophobic monomer-derived units is about 0.1 mole% or more. In another embodiment it is about 0.5 mole% or more and in a further embodiment it is about 1 mole% or more of all the monomer units comprised in the polymer.

In another embodiment of the invention the pendant hydrocarbyl group comprises an alkyl function with at most 11 carbon atoms and the hydrophobically modified polymer contains, to a level, of from 0.1, such as from such as from 0.5, for example from 1, to 20, such as to 10, for example to 5 mole%, based on the amount of monomer units in the polymer, monomeric units connected to such pendant hydrocarbyl group.

The pendant hydrocarbyl group is connected to the backbone of the hydrophobically modified polymer by via a non-ester containing linking group. Non-ester containing linking groups may be a direct bond or:

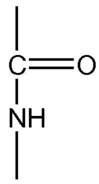
or
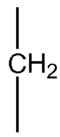
or
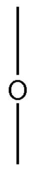

or

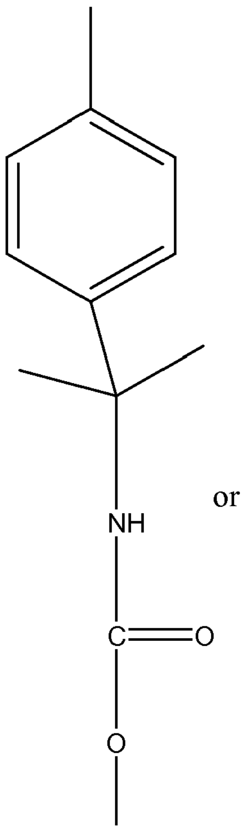
or
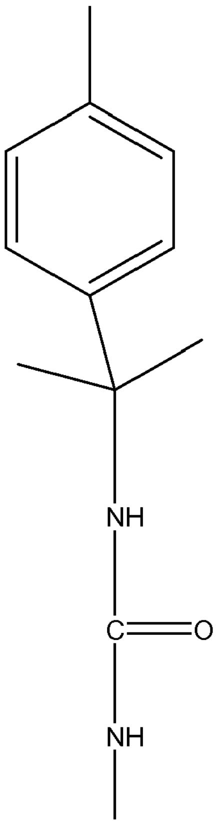
or
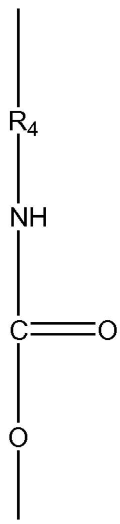
or
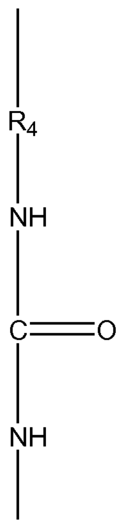

wherein $R_4$ is a hydrocarbylene group having 1 to 10 carbon atoms, preferably $CH_2$, and the top bond of the linking group is connected to the polymer backbone and the bottom bond is connected to the pendant hydrocarbyl group. Preferably, the non-ester containing linking group is a, but not limited to, urea, urethane, imide, or amide containing linking group, more preferably a urea or urethane containing linking group.

The polymers of the invention can be produced by copolymerizing suitable monomers, as described above, but they can also be produced by modification of a polymer. Hence in an embodiment of the invention, the polymer is produced in a conventional way by reacting a functional polymer with a hydrophobic modification agent, such as reacting a copolymer of maleic anhydride, including polyisobutylene succinic acid copolymers (PIBSA), with a fatty amine.

Alternatively, the hydrophobically modified polymer may be produced by reacting a functional polymer with a hydrophobic modification agent.

The hydrophobically modified polymer is suitably free from, or contains at most 1, preferably at most 0.1, more preferably at most 0.01 mole% based on the amount of monomer units in the polymer, of monomeric units connected to pendant hydrocarbyl group having at least 10, preferably at least 8, more preferably at least 6 carbon atoms connected to the backbone of said hydrophobically modified polymer via an ester containing linking group.

The hydrophobically modified polymer may obtained by copolymerizing at least a first and at least a second ethylenically unsaturated monomer, wherein said first monomer is an ethylenically unsaturated monomer with a hydrocarbyl group having from 6, preferably from 8, more preferably from 11 to 40, preferably to 32, more preferably to 24 carbon atoms being connected to the unsaturated function of said monomer via a non-ester containing linkage, preferably a urea, urethane, imide and amide containing linkage, more preferably a urea or urethane containing linkage; and said second monomer is an ethylenically unsaturated monomer free from hydrocarbyl groups having at least 11, preferably at least 8, more preferably at least 6 carbon atoms connected to the unsaturated function of the monomer. The first and second monomers are present in a molar ratio of from 0.1:99.9 to 90:10.

When the hydrocarbyl group has at least 12 carbon atoms, the first and second monomers are usually present in a molar ratio of from 0.1:99.9 to 20:80; preferably from 0.5:99.5 to 10:90, more preferably from 1:99 to 5:95.

When the hydrocarbyl group has at most 11 carbon atoms, the first and second monomers are usually present in a mutual molar ratio of from 1:99 to 90:10; preferably from 5:95 to 70:30, more preferably from 10:90 to 50:50. In another embodiment the hydrophobically-modified polymer is obtained by first copolymerizing a monomer mixture comprising at least a hydrophobic and a hydrophilic monomer and reacting the resulting copolymer with further hydrophilic or hydrophobic reagents as to refine its properties.

Monomers with a hydrocarbyl group having from 6 to 40 carbon atoms connected to the unsaturated function thereof via a non-ester containing linkage (herein also referred to hydrophobe-bearing monomers) include those with the following structure (VIIIII)

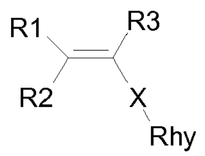

(VIIIII)

where R1, R2, and R3 are independently selected from H, $CH_3$, COOH, and $CH_2COOH$,

X is a direct bond or

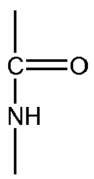 or 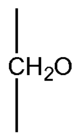 or 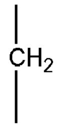 or 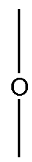

or

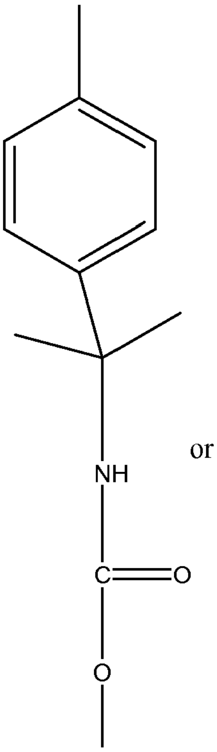

or

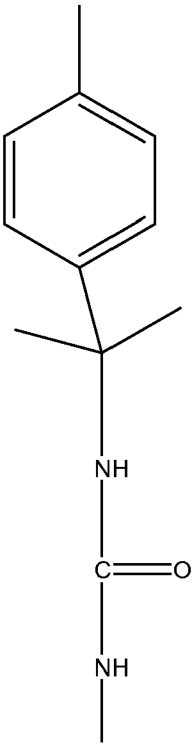

or

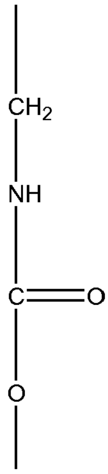

or

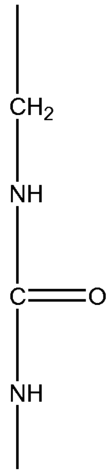

wherein $R_4$ is a hydrocarbylene group having from 1 to 10 carbon atoms, preferably $CH_2$, the top bond of X is connected to the double bond in (VIIII) and the bottom bond of X is connected to $R_{hy}$, and $R_{hy}$ is the hydrocarbyl group having from 6, preferably from 8, more preferably form 11 to 40, preferably to 32, more preferably to 24 carbon atoms.

Hydrophobe-bearing monomers of the above type are commercially available or can be obtained by methods well known in the art, for example by reacting a ethylenically unsaturated isocyanate, such as allyl-isocyanate or 3-isopropyl-benzyl-$\alpha$,$\alpha$-dimethylisocyanate, with an alcohol or amine containing a hydrocarbyl group having from 6 to 40 carbon atoms, by reacting an ethylenically unsaturated acid monomer, such as acrylic acid, with an amine containing a hydrocarbyl group having from 6 to 40 carbon atoms. Other methods to synthesize such monomers are well known to the person skilled in the art of organic synthesis.

Examples of hydrophobe-bearing monomers where the hydrophobe is linked to the double bond of the monomer include t-octyl acrylamide, n-octyl acrylamide, lauryl acrylamide, stearyl acrylamide, behenyl acrylamide, 1-allyl naphthalene, 2-allyl naphthalene,1-vinyl naphthalene, 2-vinyl naphthalene, styrene, $\alpha$-methyl styrene, 3-methyl styrene, 4-propyl styrene, t-butyl styrene, 4-cyclohexyl styrene, 4-dodecyl styrene, 2-ethyl-4-benzyl styrene and 4-(phenyl butyl) styrene.

The ethylenically unsaturated monomer free from hydrocarbyl groups having 11 or more, preferably 8 or more, more preferably 6 or more carbon atoms connected to the unsaturated function of the monomer, i.e. the second monomer, may be anionic ethylenically unsaturated monomers, cationic ethylenically unsaturated monomers, non-ionically ethylenically unsaturated monomers, zwitterionic ethylenically unsaturated monomers, mixtures thereof and salts thereof.

In an embodiment, the hydrophobically modified polymer is anionic and is synthesized from at least one first ethylenically unsaturated hydrophobe-bearing monomer and at least one second ethylenically unsaturated monomer that is anionic and referred to as an anionic ethylenically unsaturated monomer hereforth. In another embodiment, the hydrophobically modified polymer is cationic and is synthesized from at least one first ethylenically unsaturated hydrophobe-bearing monomer and and at least one second ethylenically unsaturated monomer that is cationic and referred to as a cationic ethylenically unsaturated monomer hereforth. In yet another embodiment, a the hydrophobically modified polymer is non-ionic and is synthesized from at least one first ethylenically unsaturated hydrophobe-bearing monomer and at least one second ethylenically unsaturated monomer that is non-ionic and referred to as a non-ionic ethylenically unsaturated monomer hereforth. In a further embodiment, a the hydrophobically modified polymer is zwitterionic and is synthesized from at least one first ethylenically unsaturated hydrophobe-bearing monomer and at least one second ethylenically unsaturated monomer that is zwitterionic and referred to as a zwitterionic ethylenically unsaturated monomer hereforth. In this embodiment, the polymer contains positive and negative charges which are on the same monomer repeat unit. In yet another embodiment, the hydrophobically modified polymer is zwitterionic and synthesized from at

least one first ethylenically unsaturated hydrophobe-bearing monomer and at least one anionic ethylenically unsaturated second monomer and at least one cationic ethylenically unsaturated second monomer and at least one hydrophobe-bearing monomer. In this embodiment, the polymer contains positive and negative charges which are on different monomer repeat units.

Herein an anionic ethylenically unsaturated monomer is defined as any monomer that is capable of introducing a negative charge to the hydrophobically modified polymer. These anionic ethylenically unsaturated monomers include of acrylic acid, methacrylic acid, ethacrylic acid, α-chloro-acrylic acid, α-cyano acrylic acid, β-methyl-acrylic acid (crotonic acid), α-phenyl acrylic acid, β-acryloxy propionic acid, sorbic acid, α-chloro sorbic acid, angelic acid, cinnamic acid, p-chloro cinnamic acid, β-styryl acrylic acid (1-carboxy-4-phenyl butadiene-1,3), itaconic acid, maleic acid, citraconic acid, mesaconic acid, glutaconic acid, aconitic acid, fumaric acid, tricarboxy ethylene, 2-acryloxypropionic acid, 2-acrylamido-2-methyl propane sulfonic acid (AMPS), vinyl sulfonic acid, sodium methallyl sulfonate, sulfonated styrene, allyloxybenzene sulfonic acid and their salts. The preferred salts of hydrophilic acid monomers are sodium, potassium or ammonium salts. Moieties such as maleic anhydride or acrylamide that can be derivatized to an acid-containing group can be used. Combinations of acid-containing hydrophilic monomers can also be used. In one aspect the acid-containing hydrophilic monomer is acrylic acid, maleic acid, itaconic acid, methacrylic acid, 2-acrylamido-2-methyl propane sulfonic acid , vinyl sulfonic acid, sodium methallyl sulfonate, sulfonated styrene, allyloxybenzene sulfonic acid or mixtures thereof and their salts.

Cationic polymers can be obtained by (co)polymerizing a cationic monomer or by functionalizing a polymer after polymerization. Cationic ethylenically unsaturated monomers are ethylenically unsaturated monomers which are capable of introducing a positive charge to the hydrophobically-modified copolymer. In an embodiment of the present invention, the cationic ethylenically unsaturated monomer has at least one amine functionality. The cations in the polymer may be obtained by forming amine salts of all or a portion of the amine functionality, and/or by quaternizing all or a portion of the amine functionality to form quaternary ammonium salts, or by oxidizing all or a portion of the amine functionality to form N-oxide groups. As used herein, the term "amine salt" means that the nitrogen atom of the amine functionality is covalently bonded to from one to three organic groups and from three to one protons, such that there are 4 bonds to the nitrogen and it is associated with an anion. As used herein, the term "quaternary ammonium salt" means that a nitrogen atom of the amine functionality is covalently bonded to four organic groups and is associated with an anion.

Monomers that can form cations include, but are not limited to, N,N-dialkylaminoalkyl (meth)acrylate, N-alkylaminoalkyl (meth)acrylate, N,N-dialkylaminoalkyl (meth)acrylamide and N-alkylaminoalkyl (meth)acrylamide, where the alkyl groups are independently $C_{1-18}$ cyclic compounds such as 1-vinyl imidazole and the like. Aromatic amine containing monomers such as vinyl pyridine may also be used. Furthermore, monomers such as vinyl formamide, vinyl acetamide and the like which generate amine moieties on hydrolysis may also be used. Preferably the cationic ethylenically unsaturated monomer is N,N-dimethylaminoethyl methacrylate, tert-butyl aminoethyl methacrylate and N,N-dimethylaminopropyl methacrylamide. Cationic ethylenically unsaturated monomers that may be used include the quarternized derivatives of the above monomers as well as diallyldimethylammonium chloride also known as dimethyldiallylammonium chloride, (meth)acrylamidopropyl trimethylammonium chloride, 2-(meth)acryloyloxy ethyl trimethyl ammonium chloride, 2-(meth)acryloyloxy ethyl trimethyl ammonium methyl sulfate, 2-(meth)acryloyloxyethyltrimethyl ammonium chloride, N,N-Dimethylaminoethyl (meth)acrylate methyl chloride quaternary, methacryloyloxy ethyl betaine as well as other betaines and sulfobetaines, 2-(meth)acryloyloxy ethyl dimethyl ammonium hydrochloride, 3-(meth)acryloyloxy ethyl dimethyl ammonium hydroacetate, 2-(meth)acryloyloxy ethyl dimethyl cetyl ammonium chloride, 2-(meth)acryloyloxy ethyl diphenyl ammonium chloride and others.

As used herein, the term "nonionic ethylenically unsaturated monomer" means an ethylenically unsaturated monomer which does not introduce a charge to the hydrophobically-modified copolymer. These nonionic ethylenically unsaturated monomers include, but are not limited to, acrylamide, styrene, acylonitrile rmethacrylamide, N-alkyl(meth)acrylamide, N,N-dialkyl(meth)acrylamide such as N,N-dimethylacrylamide, hydroxyalkyl(meth)acrylates, alkyl(meth)acrylates such as, methylacrylate and methylmethacrylate, vinyl acetate, acrylonitrile, vinyl morpholine, vinyl pyrrolidone, vinyl caprolactum, ethoxylated alkyl, alkaryl or aryl monomers such as methoxypolyethylene glycol (meth)acrylate, allyl glycidyl ether, allyl alcohol, glycerol (meth)acrylate, monomers containing silane, silanol and siloxane functionalities and others. In an embodiment the non-ionic hydrophobically modified polymer contains vinyl alcohol which is typically generated by hydrolysis of vinyl acetate after the hydrophobically modified polymer has been formed. The nonionic ethylenically unsaturated monomer is preferably water soluble.

As used herein, the term "zwitterionic ethylenically unsaturated monomer" means an ethylenically unsaturated monomer which introduces both a positive and a negative charge in the same monomer repeat unit of the hydrophobically modified copolymer.

The zwitterionic ethylenically unsaturated monomers include amine oxides carboxybetaine, sulfobetaine, and phosphobetaine monomers. Examples of amine oxides include, but are not limited to, vinyl pyridine-N-oxide and tert-butyl-aminoethylmethacrylate-N-oxide. It is understood that the monomer, say N-vinyl pyridine, can be copolymerized and then the pyridine moiety is oxidized to the amine oxide. Examples of carboxybetaine monomers include, but are not

limited to, N,N'-dimethyl-N-methacryloyloxyethyl-N-(2-carboxyethyl) ammonium, (2-(2-acrylamido-2-methylpropyldimethylammonio) ethanoate, 6-(2-acrylamido-2-methylpropyl dimethylammonio) hexanoate, 4-(N,N-diallyl-N-methylammonio) butanoate, and others. Examples of sulfobetaine monomers include, but are not limited to, sulfopropyldimethylammonioethyl methacrylate, sulfoethyldimethyl-ammonioethyl methacrylate, sulfobutyldimethylammonioethyl methacrylate, sulfohydroxy propyldimethylammonioethyl methacrylate, sulfopropyldimethyl ammoniopropylacrylamide, sulfopropyldimethylammonio-propylmethacrylamide, sulfohydroxypropyldimethyl-ammoniopropylmethacryl-amide, sulfopropyldimethylammonioethyl acrylate, sulfopropyldiethyl-ammonio-ethoxyethyl methacrylate, 2-vinyl-I-(3-sulfopropyl) pyridinium betaine, 4-vinyl-I-(3-sulfopropyl) pyridinium betaine, I-vinyl-3-(3sulfopropyl) imidazolium betaine, sulfopropylmethyldiallylammonium betaine, 3-(N,N-diallyl-N-methylammonio) propanesulfonate, and others.

As mentioned before, a zwitterionic hydrophobically modified polymer can be synthesized by copolymerizing an anionic ethylenically unsaturated monomer and a cationic ethylenically unsaturated monomer with a hydrophobe-bearing monomer. Any combination of anionic and cationic monomers may be used. However, the preferred anionic monomer will introduce a sulfonate group to the copolymer.

A polymer of the present invention may comprise further monomers in addition to those mentioned above.

It should be noted that the hydrophobically modified polymers of the invention are not conventional thickeners, due to their low molecular weight. In an embodiment of the invention, the number average molecular weight of the polymers, neutralized or not, may for example be chosen such that they do not thicken a 4 wt% KCl solution in water when used at concentrations of 2 wt% in said KCl solution, at a temperature of 25°C. In another embodiment, the molecular weight of the polymer is chosen such that an aqueous solution of 2 wt% of the polymer and 4 wt% of KCl has a viscosity of 100 or less, preferably 50 or less, most preferably 16 mPas or less at a shear rate of 100 $sec^{-1}$ and a temperature of 25°C. Optionally, the polymers are neutralized.

Because of the exceptional properties observed for the compositions of the invention with the viscoelastic surfactant and the specific polymer, it is possible to reduce the amount of surfactant and/or polymer. It was also surprisingly found that the increased viscosity can be achieved at a polymer concentration that is lower than the overlap concentration c*. The polymer overlap concentration c* is a threshold concentration when polymer coils begin to densely pack in a solvent. In a dilute polymer solution where polymer concentration is below c*, the polymer coils are separated. In a polymer solution where the polymer concentration is above c*, the polymer coils are densely packed. The detailed definition of c* is described by Pierre-Gilles de Gennes in "Scaling Concept on Polymer Physics", pp. 76-77, Cornell University Press, Ithaca and London (1979). c* is measured by the plot of viscosity versus concentration. At low concentrations, the plot will follow a linear path and once the c* is reached, the slope of the line drastically increases. For the purposes herein, the polymer overlap concentration c* is measured at 25°C at atmospheric pressure in the solvent. Accordingly, in an embodiment it may be desired for economic and environmental reasons to use the polymers of the invention at a polymer concentration that is below the overlap concentration c*. Further, it is noted that the polymers of the invention were found to interact with viscoelastic surfactants in such a way that the combined use leads to increased viscosities at high temperature (tested up to 100°C) and pressures (tested up to 25 bara.)

If the combination of viscoelastic surfactant and polymer of the invention is supplied in a concentrated form, it is preferably in an aqueous, essentially salt-free form. Such an aqueous concentrate has the advantage of having a low viscosity and related easy dilution. Typical aqueous concentrates comprise one or more glycols, such as propylene glycol, so that the viscoelastic surfactant is more easily dissolved in the concentrate. Typically, the amount of viscoelastic surfactant in such a concentrate is within the range of greater than 10 %w/w, preferably 10-60 %w/w, whereas the amount of polymer ranges from about 5-30 %w/w, based on the weight of the concentrate. By the term essentially salt-free it is meant that the salt concentration is less than 0.01 %w/w, otherwise the viscosity becomes unacceptably high.

For personal care applications, the viscoelastic surfactant is used in an amount below about 40 %w/w, preferentially, below about 20 %w/w of the final viscoelastic aqueous composition. Also the viscoelastic surfactant is suitably used in an amount of about 1 %w/w or more. In an embodiment it is about 5 %w/w or more, while in another embodiment it is about 7%w/w or more, all being based on the weight of the total viscoelastic aqueous composition.

In general, the hydrophobically modified polymer is used in an amount of 10 %w/w or less. In an embodiment of the invention, it is 5 %w/w or less. In another embodiment it is 2% or less, while in a further embodiment it is 1 %w/w or less. The polymer is to be used in an amount of at least 0.01 %w/w. All of these amounts are based on the weight of the total viscoelastic aqueous composition.

The weight ratio of hydrophobically modified polymer to viscoelastic surfactant is usually from 0.1:100, preferably from 1:100, more preferably from 3:100, to 100:50, preferably to 100:100, more preferably to 50:100.The viscoelastic compositions of the invention may also comprise one or more chelating agents. Particularly when the aqueous formulations or the area where the compositions are used has a lot of hardness ions, the use of chelating agents for such ions was found to be beneficial. Without wishing to be bound by theory, it is thought that these hardness ions tend to precipitate the hydrophobically modified polymer in the aqueous solution. The addition of chelating agents, according to said theory, prevents the precipitation of these hydrophobically modified polymers and preserves the performance of the mixture of these polymers and viscoelastic surfactants in high hardness aqueous solutions.

For purposes of this invention, a chelating agent is described as any material that will chelate hardness ions, such as calcium and magnesium, in aqueous solutions. Chelating agents include but are not limited to (S,S)-ethylenediaminesuccinic acid trisodium salt, N,N-bis(carboxymethyl)-L-glutamic acid tetrasodium salt, L-aspartate-(N,N)-diacetic acid tetrasodium salt, N-2-hydroxyethyliminodiacetic acid disodium salt, methylglycinediacetic acid trisodium salt, ethylenediaminetetraacetic acid, nitorilotriacetic acid, diethylenetriaminepentaacetic acid, hydroxyethylethylenediaminetriacetic acid, triethylenetetraminehexaacetic acid, 1,3-propanediaminetetraacetic acid, 1,3-diamino-2-hydroxypropanetetraacetic acid, dihydroxyethylglycine, glycol ether diamine tetraacetic acid, hydroxyethanediphosphonic acid, aminotrimethylenephosphonic acid, 1,2,4-butanetricarboxylic acid, dihydroxyethylethylenediaminediacetic acid, sodium gluconate, sodium glucoheptonate, inositol hexaphosphate, hydroxyethanoic acid, 2-hydroxypropanoic acid, 2-hydroxysuccinic acid, 2,3-dihydroxybutanedioic acid, and 2-hydroxy-1,2,3-propanetricarboxylic acid and their salts. The preferred chelating agents are aminocarboxylates, such as (S,S)-ethylenediaminesuccinic acid trisodium salt, N,N-bis(carboxymethyl)-L-glutamic acid tetrasodium salt, L-aspartate-(N,N)-diacetic acid tetrasodium salt, N-2-hydroxyethyliminodiacetic acid disodium salt, methylglycinediacetic acid trisodium salt, ethylenediaminetetraacetic acid, nitrilotriacetic acid, and their salts. For purposes of this invention, a high hardness aqueous solution is defined as a solution with a hardness of greater than 100 ppm expressed as $CaCO_3$, more preferably greater than 250 ppm as $CaCO_3$, and most preferably greater than 500 ppm as $CaCO_3$.

In addition to the aqueous viscoelastic solution, the personal care compositions of the present invention also include a personal care active ingredient. In hair care formulations, the personal care active ingredients may provide beneficial properties to hair such as improved cleanliness, fragrance, improved shine, improved wet combability, improved dry combability, improved wet feel, improved dry feel, improved strength, or improved color. In skin care formulations, the personal care active ingredients may provide beneficial properties to skin such as improved cleanliness, fragrance, preparation for shaving, improved smoothness, improved appearance, reduced wrinkle, improved color, or improved tone. Such active ingredients include, without limitation, thickeners, emulsifiers, aesthetic modifiers, UV filters, humectants (such as hydroxyethyl urea, available from Akzo Nobel Surface Chemistry under the trademark HYDROVANCE®), lubricants, skin whitening ingredients, silicones, powders, de-viscosifying agents, moisturizers, emollients, solvents, chelating agents, vitamins, antioxidants, botanical extracts, pH adjusting agents, preservatives, fragrances, waterproofing agents, anti-aging agents, firming forming polymers, toning agents, dyes, pigments, colors, polymers, conditioning agents, rheology modifiers, surfactants, opacifiers, foaming agents, heat generating agents and/or effervescing agents, glitter and decorative beads and shapes. In an embodiment of the invention, the personal care composition may be a hair care formulation, such as a shampoo or a conditioner, or a skin care formulation such as a body wash or shave preparation.

The active ingredient will be present in the personal care composition from about 0.01 to about 10% of the total formula weight. In an embodiment of this invention the active ingredient will be present from about 0.1 to about 5 percent by weight based on the total weight of the personal care formulation (dry basis). In another embodiment, the active viscoelastic aqueous composition will be present from about 0.3 to 4% of the total weight of personal care formulation (dry basis).

The viscoelastic aqueous composition will be present from about 0.1 to about 10wt% of the total personal care formulation (dry basis). In an embodiment of this invention the viscoelastic aqueous composition will be present from about 0.2 to about 5wt% of the total personal care formulation (dry basis). In another embodiment, the viscoelastic aqueous composition will be present from about 0.3 to 4wt% of the total weight of the personal care formulation (dry basis).

In an embodiment, salt can be added to the formulation to assist in increasing viscosity. Typical levels of salt that can be added to the personal care composition will be from about 0.1 to about 10% based on the total weight of the composition. Typical salts will be, for example, alkaline metal and alkaline earth cations of common anions (e.g. sulfate, chloride, bromide, nitrate, phosphate, carbonate, and mixtures thereof). In another embodiment the salt will be sodium chloride, calcium chloride or mixtures thereof. In yet another embodiment the salt will be present in an amount from about 1 to about 5 percent based on the total weight of the composition.

With the exception of the information in the examples, or where otherwise indicated, all numbers or expressions referring to quantities of ingredients, reaction conditions, and the like, used in the specification and claims are to be understood as modified in all instances by the term "about". Further, where numerical ranges are disclosed they are meant to be continuous ranges that include every value between the minimum and maximum value as presented. Wt% and %w/w mean percent by weight.

The invention will now be further described in connection with the following Examples which are not intended to limit the scope thereof. Unless otherwise stated, all parts and percentages refer to parts and percentages by weight. All numbers given relate to the amount of active material. So if in the examples 10 %w/w of a chemical is specified, then the amount to be used of the supplied product is to be increased if the product is supplied in a diluted form.

EXAMPLES

Except where indicated otherwise, the viscosity of samples has been determined over a broad shear rate range using a stress-controlled rheometer SR-5000 (from Rheometric Scientific, which is now TA Instruments). The sample was placed between two circular parallel plates of 25 mm or 40 mm in diameter and evaluated at a temperature of 25 °C. Typically the initial stress was 0.5 Pa and the final stress was in the 150-400 Pa range, depending on the viscosity of the sample, with the lower final stress selected for samples with lower viscosity. In the linear sweep mode a stress increment of 0.5-2 Pa was applied.

Aromox® APA-TW is a commercial tallowalkylamidopropyl dimethyl amine oxide supplied by AkzoNobel.

POLYFLOS® HM 21 is a hydrophobically modified hydroxypropyl guar gum supplied by Lamberti spa, and was used as received. This polymer was used as a comparison for the polymers of the invention.

**Preparation of hydrophobically-modified polymer R7-33-43**

Synthesis of behenyl alcohol urethane of m-TMI monomer:

75 g of behenyl alcohol (available from Cognis) was melted and added to a reactor and heated to 95°C and sparged with nitrogen for 4 hours to remove any water. The nitrogen sparge was discontinued and the reaction temperature lowered to 78°C. 0.3 g of monomethyl ether hydroquinone (MEHQ) inhibitor and 0.3 grams of Stannous 2-ethylhexanoate (FASCAT® 2003 available from Arkema Inc, Philadelphia, PA) were then added to the reactor. 47.6 g of 3-isopropenyl-$\alpha,\alpha$-dimethylbenzyl isocyanate (m-TMI available from Cytec, Stamford, CT) was then slow added to the reactor over a period of 30 minutes. A slight exotherm was observed which raised the temperature to 78 to 83°C. After the addition, the reactor was held at 80°C for an additional 30 minutes. The final product was a liquid which cooled down to a solid at room temperature.

Synthesis of the polymer R7-33-43:

An initial charge of 40.8 g of water and 153.5 g of isopropyl alcohol were added to a 1 liter glass reactor. The reactor contents were heated to reflux (approximately 82 to 84°C). In a separate beaker, 142 g of acrylic acid was warmed to 55°C and then 60 g of the behenyl alcohol urethane of m-TMI of the previous step was added with stirring. This warm mixture was added to the reactor at reflux over a period of 2.5 hours. A solution of 1.9 g of sodium persulfate dissolved in 60 g of water was simultaneously added but over said period of 2.5 hours. The reaction temperature was maintained at about 85°C for one hour. A scavenge feed (to minimize residual monomer) containing 0.175 g of sodium persulfate dissolved in 10 g of water was then added to the reactor over 30 minutes and maintained for an additional 30 minutes. The reactor was then set up for distillation which was carried out at increased temperature and/or reduced pressure, to ensure a controlled distillation without polymer degradation. A small amount of ANTIFOAM® 1400 (0.12 g) (from Dow Chemical) was added to suppress any foam generated during distillation. The alcohol (the cosolvent) was removed from the polymer solution by azeotropic distillation. During the distillation, about 1350 g of water was added. Approximately, 263 g of a mixture of water and isopropyl alcohol were distilled off. After distillation was completed, the reaction mixture was cooled and 21.8 g of 50% NaOH was added. The final product had a pH of 2.2 and solids of 13.3 percent.

**Preparation of hydrophobically-modified polymer R7-33-61**

Synthesis of Armeen 18D m-TMI monomer:

70 g of octadecylamine (Armeen® 18D available from AkzoNobel Surface Chemistry) was melted and added to a reactor and heated to 90°C. The liquid octadecylamine was sparged with nitrogen for 4 hours to remove any water in the material. The nitrogen sparge was discontinued and the reaction temperature lowered to 78°C. 0.3 g of monomethyl ether hydroquinone (MEHQ) inhibitor and 0.3 grams of Stannous 2-ethylhexanoate (FASCAT® 2003 available from Arkema Inc, Philadelphia, PA) were then added to the reactor. 52.2 g of 3-isopropenyl-$\alpha,\alpha$-dimethylbenzyl isocyanate (m-TMI available from Cytec, Stamford, CT) was then slowly added to the reactor over a period of 45 minutes. A slight exotherm was observed which raised the temperature to 80 to 83°C. After the addition, the reactor was held at 80°C for an additional 30 minutes. The final product was a liquid which cooled down to a solid at room temperature.

Synthesis of the polymer R7-33-61:

An initial charge of 38 g of water and 150 g of isopropyl alcohol were added to a 1 liter glass reactor. The reactor

contents were heated to reflux (approximately 82 to 84°C). In a separate beaker, 142 g of acrylic acid was warmed to 55°C and then 58.4 g of the Armeen 18D m-TMI monomer was added with stirring. This warm mixture was added to the reactor at reflux over a period of 2.5 hours. A solution of 1.9 g of sodium persulfate dissolved in 61 g of water was simultaneously added but over a period of 2.5 hours. The reaction temperature was maintained at about 85°C for one hour. A scavenge feed containing 0.17 g of sodium persulfate dissolved in 10 g of water was then added to the reactor over 30 minutes and held at temperature for an additional 30 minutes. The reactor was then set up for distillation. A small amount of ANTIFOAM® 1400 (0.12 g) (from Dow Chemical) was added to suppress any foam generated during distillation. The alcohol cosolvent was removed from the polymer solution by azeotropic distillation. During the distillation, about 1080 g of water was added. Approximately, 251 g of a mixture of water and isopropyl alcohol were distilled off. After distillation was completed, the reaction mixture was cooled. The final product had a pH of 2.5 and solids of 15.7 percent.

**Preparation of hydrophobically-modified polymer R7-36-72**

Synthesis of 2-decyl-tetradecanol m-TMI monomer:

150 g of 2-decyl-tetradecanol (branched alcohol) [Isofol® 24 (97.5%) (available from Sasol, Houston, TX)] was added to a 500 ml reactor and heated to 80°C. The reactor contents were sparged with nitrogen for 4 hours to remove any water in the material. The nitrogen sparge was discontinued and the reaction temperature lowered to 68°C. 0.33 g of monomethyl ether hydroquinone (MEHQ) inhibitor and 0.33 g of Stannous 2-ethylhexanoate (FASCAT 2003 available from Arkema Inc, Philadelphia, PA) were then added to the reactor. 82.5 g of 3-isopropenyl-$\alpha$,$\alpha$-dimethylbenzyl isocyanate (m-TMI available from Cytec, Stamford, CT) was then slowly added to the reactor over a period of 30 minutes. A slight exotherm was observed which raised the temperature to 70 to 71 °C. After the addition, the reactor was held at 72°C for an additional 60 minutes. The final product was a liquid.

Synthesis of the polymer R7-36-72:

An initial charge of 43 g of water and 133 g of isopropyl alcohol were added to a 1 liter glass reactor. The reactor contents were heated to reflux (approximately 82 to 84°C). A first monomer solution of 33 g of acrylic acid, 20.11 g of 2-decyl-tetradecanol m-TMI monomer (synthesized above), 9,9 g of isopropyl alcohol and 4.1 g of hydroxypropyl methacrylate was added to the reactor at reflux over a period of 75 minutes. A second monomer solution containing 12.8 grams of 50% 2-acrylamido-2-methyl propane sulfonic acid, sodium salt in 20 g of water was added concurrently over a period of 75 minutes. An initiator solution of 0.97 g of sodium persulfate dissolved in 28.3 g of water was simultaneously added but over a period of 90 minutes. The reaction temperature was maintained at about 85°C for one hour. A scavenge feed containing 0.15 g of sodium persulfate dissolved in 10 g of water was then added to the reactor over 30 minutes and the temperature was held for an additional 30 minutes. The reactor was then set up for distillation and a small amount of ANTIFOAM® 1400 (0.12 g) (from Dow Chemical) was added to suppress any foam generated during distillation. The alcohol cosolvent was removed from the polymer solution by azeotropic distillation. Approximately, 185 g of a mixture of water and isopropyl alcohol were distilled off. During the distillation, about 242 g of water was added to replace the distillate and keep the viscosity at a manageable level. After distillation was completed, the reaction mixture was cooled and 7 g of 50% NaOH in 15 g of water was added. The final product had a pH of 4.2 and solids of 16.9 percent.

**Preparation of polymer R7-33-158**

An initial charge of 77 g of water and 100 g of isopropanol were added to a 1 liter glass reactor. The reactor contents were heated to 82°C. A first solution which is a mixture of 72.7 g of acrylic acid and 21.6 g of an N-octadecyl acrylamide dissolved in 50 g of isopropanol was added to the reactor over a period of 80 minutes. A second solution of 0.97 g of sodium persulfate dissolved in 46 g of water was simultaneously added at the same time but over a period of 90 minutes. After the second solution addition was completed, a solution of 0.09 g of sodium persulfate dissolved in 14 g of water was then added over a period of 10 minutes. The reactor was then set up for distillation. The alcohol cosolvent was removed from the polymer solution by azeotropic distillation. During the distillation, 450 g of water was dripped in and approximately, 303 g of a mixture of water and alcohol were distilled off. The final product was a clear colorless viscous solution with a pH of 2.6 and a solids content of 19.9%.

**Preparation of polymer R7-33-28**

An initial charge of 72.9 g of water and 50.9 g of ethanol were added to a 500 ml glass reactor. The reactor contents were heated to reflux (approximately 82 to 84°C). A mixture of 38.1 g of acrylic acid and 5.55 g of lauryl

methacrylate was added to the reactor at reflux over a period of 3 hours. A solution of 0.6 g of sodium persulfate dissolved in 32 g of water was simultaneously added but over a period of 4 hours. The reaction temperature was maintained at about 85°C for 30 minutes. The reactor was then set up for distillation. The alcohol cosolvent was removed from the polymer solution by azeotropic distillation. During the distillation, 38.8 g of 50% NaOH dissolved in 70 g of water was dripped in added. Approximately, 99.3 g of a mixture of water and ethanol were distilled off. The final product has 25.25% solid and pH = 7.5.

**Preparation of Polymer J3-9-46**

An initial charge of 150 g diallyldimethylammonium chloride (65 % Aldrich commercial material further concentrated to 88 % by removal of water), 150 g isopropyl alcohol, and 32.2 g 2-decyl-tetradecanol m-TMI monomer (synthesized above) was added to a 1 liter glass reactor fitted with a condenser for reflux. An initiator feed consisting of Esperox 28 in isopropyl alcohol (16.4 g in a total volume of 100 mL) was prepared. The reaction was maintained in the range of 83 to 87 °C, to allow for reflux of IPA, while the initiator solution was added over a period of 2 hr. Following the initiator slow addition, the reaction was maintained at 83 °C (refluxing IPA) for 2.5 hours. The reaction was cooled below the reflux temperature, and the reactor was then fitted with a Dean-Stark trap to allow for collection and removal of distillate. IPA/water was removed from 81 to 86 °C while the reaction volume was replenished with water from an addition funnel to maintain an acceptable viscosity. Total distillate collection up to this point was 194 g, while the added water was 350 g. Because of intense foaming, 195 g additional water was added to the reaction, and the reaction mixture was then transferred to a Roto-Vap apparatus, where an additional 110 g of distillate was removed under vacuum at 55 °C. The weight of the final material after being subjected to distillation was 690 g. This was a pink-tinted, lightly cloudy liquid with a pH of 4 and consisting of 23 % active polymer by weight.

**Table 1: Analysis of the hydrophobically modified polymers compared with the conventional POLYFLOS® HM 21.**

| | R7-33-43 | R7-33-61 | R7-36-72 | R7-33-28 | R7-33-158 | POLYFLOS® HM 21 |
|---|---|---|---|---|---|---|
| **$M_w$ (Da)** | 16131 | 19775 | 8459 | 29956 | 19528 | 537550 |
| **$M_n$ (Da)** | 2735 | 3199 | 1488 | 4095 | 2750 | 13641 |
| **Dispersity = $M_w/M_n$** | 5.9 | 6.2 | 5.7 | 7.3 | 7.1 | 39.4 |

**Example 1 and Comparative examples A-B:** Rheology of Aromox APA-TW and hydrophobically modified polymer R7-33-43

Samples A, B, and 1 were made based on the amount shown in Table 2.

**Table 2: Sample Preparation of Aromox APA-TW + R7-33-43**

| Ex. | Description | Wt of Polymer (g) | Wt. of Aromox APA-TW (g) | Wt of 4% KCl Solution (g) | pH |
|---|---|---|---|---|---|
| A | 3wt% (active) Aromox APA-TW, no polymer, in 4% KCl | None | 2.1270 | 27.9093 | 9.72 |
| B | 0.2wt% (active) R7-33-43, no surfactant, in 4% KCl | 0.4956 | None | 29.7272 | 11.56 |
| 1 | 3wt% (active) Aromox APA-TW + 0.2wt% (active) R7-33-43, in 4% KCl | 0.4502 | 2.0939 | 27.5104 | 10.30 |

For samples A, B and 1, a strain-controlled rheometer ARES (from Rheometric Scientific, which is now TA Instruments) was used to conduct the Steady Strain Rate Sweep at 25°C, with an initial strain rate of 0.01 $s^{-1}$ and a final strain rate of 100 $s^{-1}$. Data was collected, 10 data points per strain rate decade. Parallel plates of diameter of 25 mm were used, and temperature was controlled by peltier heating.

The rheology profile is graphed in Figure 1. Clearly sample 1 shows significantly higher viscosity than sample

A and sample B, indicating a synergistic viscosity increase achieved by combination of Aromox APA-TW and R7-33-43 polymer (a polymer with a urethane linkage) in 4% KCl. The same synergistic viscosity increase was also found at 50°C and at 80°C.

In a separate rheology test at 25°C, a dynamic frequency of $10^{-1}$ to $10^{2}$ rad/s was used and for the solution of Example 1 G' was higher than G" over the whole range, indicating the solution showed viscoelastic behavior.

**Example 2 and Comparative examples C-D** Rheology of Aromox APA-TW and hydrophobically modified polymer R7-33-61

Samples C, D, and 2 were made based on the amount shown in Table 3.

**Table 3: Sample Preparation of Aromox APA-TW + R7-33-61**

| Ex. | Description | Wt of Polymer (g) | Wt. of Aromox APA-TW (g) | Wt of 4% KCl Solution (g) | pH |
|---|---|---|---|---|---|
| C | 3wt% (active) Aromox APA-TW, no polymer, in 4% KCl | None | 2.1270 | 27.9093 | 9.72 |
| D | 0.2wt% (active) R7-33-61, no surfactant, in 4% KCl | 0.3992 | None | 29.6257 | 11.25 |
| 2 | 3wt% (active) Aromox APA-TW + 0.2wt% (active) R7-33-61, in 4% KCl | 0.3834 | 2.0971 | 27.5424 | 10.00 |

For samples C, D and 2, a strain-controlled rheometer ARES (from Rheometric Scientific, which is now TA Instruments) was used to conduct the Steady Strain Rate Sweep at 25°C, with an initial strain rate of 0.01 $s^{-1}$ and a final strain rate of 100 $s^{-1}$. Data was collected, 10 data points per strain rate decade. Parallel plates of diameter of 25 mm were used, and temperature was controlled by peltier heating.

The rheology profile is graphed in Figure 2. Again, sample 2 shows significantly higher viscosity than sample C and sample D, indicating a synergistic viscosity increase achieved by combination of Aromox APA-TW and R7-33-61 polymer (a polymer with a urea linkage) in 4% KCl. The same synergistic viscosity increase was also found at 50°C and at 80°C

In a separate rheology test at 25°C, a dynamic frequency of $10^{-1}$ to $10^{2}$ rad/s was used and for the solution of Example 2 G' was higher than G" over the whole range, indicating the solution showed viscoelastic behavior.

**Comparative examples E and F:** Rheology of Aromox® APA-TW and POLYFLOS® HM 21. Formulations were as shown in Table 4.

**Table 4: Sample Preparation of Aromox APA-TW + POLYFLOS® HM 21**

| Ex. | Description | Wt of Polymer (g) | Wt. of Aromox APA-TW (g) | Wt of 4% KCl Solution (g) | pH |
|---|---|---|---|---|---|
| E | 3wt% (active) Aromox APA-TW, no polymer, 4% KCl | None | 2.1000 | 27.9326 | 11.27 |
| F | 3wt% (active) Aromox APA-TW + 0.2wt% (active) POLYFLOS® HM 21 in 4% KCl | 0.0774 | 2.0985 | 27.8268 | 11.81 |

The rheology profile is graphed in Figure 3. The result show that samples E and F overlay very well with each other, indicating no rheological synergy upon combination of POLYFLOS® HM 21 with Aromox APA-TW in 4% KCl.

It is pointed out that the polymer in accordance with the invention (R7-33-43 of Example 1) has a much lower molecular weight than the conventional thickener POLYFLOS® HM 21 of the Comparative example F, see the Table 1 above. Hence it is surprising to see that the use of conventional hydrophobic polymers does not lead to the viscosities observed when using polymers of the invention.

**Examples 3 and 4 and Comparative example G - Rheology of Aromox APA-TW, polymer R7-33-43 and polymer R7-33-61.**

Here the performance of an amine-oxide viscoelastic surfactant in combination with the hydrophobically modified polymers R7-33-43 and R7-33-61 was investigated for an aqueous environment containing 4% KCl at elevated temperature (93°C (200°F)) and at an elevated pressure (27.5 bar (400psi)), to mimic oil-well-stimulation conditions. The amounts of surfactant and polymer used as well as the results obtained are presented in the following Table 5, with the viscosity being determined after two hours at shear rate of 100 $s^{-1}$, using a Grace M5600 rheometer at said pressure and temperature with rotor R1 and bob B5.

**Table 5 - Sample preparation of samples G, 3 and 4**

| Ex. | Description | Wt of Polymer (g) | Wt. of Aromox APA-TW (g) | Wt of KCl solution (g) | pH | HTHP Viscosity at 93°C/2hr (mPa-s) |
|---|---|---|---|---|---|---|
| G | 3wt% (active) Aromox APA-TW, no polymer, in 4% KCl | 0.0000 | 6.9683 | 93.0322 | 10.58 | 12.49 |
| 3 | 3wt% (active) Aromox APA-TW + 0.2wt% (active) R7-33-43, in 4% KCl | 1.5275 | 7.1534 | 91.5231 | 11.72 | 210.84 |
| 4 | 3wt% (active) Aromox APA-TW + 0.2wt% (active) R7-33-61, in 4% KCl | 1.2661 | 6.9709 | 91.7554 | 10.68 | 82.35 |

Clearly, also in a KCl-containing aqueous formulation and at high temperature and pressure, the combination of viscoelastic surfactant and hydrophobically modified polymer according to the invention gave a very high viscosity, even when polymer used in a small amount and despite the low molecular weight of the polymer.

**Example 5 and Comparative examples H and I - Rheology of Aromox APA-TW and polymer R-36-72**

Here, the performance of an amine-oxide viscoelastic surfactant in combination with hydrophobically modified polymer R7-36-72 was investigated for an aqueous environment containing 4%w/w KCl and an amount of $CaCl_2$ of 2,776 ppm (0.2776 %w/w) at 25°C. The amounts of surfactant and polymer used as well as the results obtained are presented in the Table 6.

**Table 6 - Sample preparation of samples H, I and 5**

| Ex. | Description | Wt. of Polymer (g) | Wt. of Aromox APA-TW (g) | Wt. of 4% KCl (g) | pH |
|---|---|---|---|---|---|
| H | 3wt% (active) Aromox APA-TW, no polymer, in brine of $CaCl_2$/KCl | 0.0000 | 2.0852 | 27.9146 | 10.91 |
| I | 0.2wt% (active) R7-36-72, no Aromox APA-TW, in brine of $CaCl_2$/KCl | 0.3714 | 0.0000 | 29.6453 | 10.26 |
| 5 | 3wt% (active) Aromox APA-TW + 0.2wt% (active) R7-36-72, in brine of $CaCl_2$/KCl | 0.3857 | 2.1083 | 27.5273 | 10.06 |

The combination in accordance with the invention gave a higher viscosity than the use of the surfactant or polymer alone, showing the synergistic behavior, despite the low molecular weight of the polymer and the small quantity in which it was used, as is demonstrated in Figure 4. Polymer R7-36-72 contained AMPS monomer that provided tolerance to Calcium brine.

**Example 6 and Comparative example J - Rheology of Aromox APA-TW and polymer R-33-43 with EDTA**

In these examples the experiment of Example 5 was repeated, except that hydrophobically modified polymer R7-33-43 was used, while also using the well-known chelate EDTA (Dissolvine NA) in the composition. Samples 6 and J were prepared according to the amount shown in Table 7.

**Table 7 - sample preparation, samples 6 and J**

| Ex. | Description | Wt. of Polymer R7-33-43 (g) | Wt. of Aromox APA-TW (g) | Wt. of Dissolvine NA (EDTA) (g) | Wt. of Brine (g) | pH |
|---|---|---|---|---|---|---|
| J | 3wt% (active) Aromox APA-TW + EDTA, no polymer, in brine of $CaCl_2$/KCl | 0.0000 | 2.0917 | 0.3356 | 27.5758 | 9.93 |
| 6 | 3wt% (active) Aromox APA-TW + 0.2wt% (active) R7-33-43 + EDTA, in brine of $CaCl_2$/KCl | 0.4624 | 2.0940 | 0.3320 | 27.1467 | 9.80 |

Figure 5 shows the viscosity of these formulations. The results show that the combination of viscoelastic surfactant and low molecular weight hydrophobically modified polymer gives exceptionally high viscosity at low concentrations also in the presence of a chelate.

**Example 7 and comparative example K - Rheology of Aromox APA-TW and polymer R7-33-158**

Samples 7 and K were prepared according to the amounts indicated in Table 8

**Table 8 - Preparation of samples 7 and K**

| Ex. | Description | Wt. of Polymer R7-33-158 (g) | Wt. of Aromox APA-TW (g) | Wt. of 4% KCl (g) | pH |
|---|---|---|---|---|---|
| K | 3wt% (active) Aromox APA-TW, no polymer, in 4% KCl | 0.0000 | 2.1270 | 27.9093 | 9.72 |
| 7 | 3wt% (active) Aromox APA-TW + 0.2wt% R7-33-158, in 4% KCl | 0.3026 | 2.1022 | 27.6135 | 12.06 |

For sample K, a strain-controlled rheometer ARES (from Rheometric Scientific, which is now TA Instruments) was used to conduct the Steady Strain Rate Sweep at 25°C, with an initial strain rate of 0.01 $s^{-1}$ and a final strain rate of 100 $s^{-1}$. Data was collected, 10 data points per strain rate decade. Parallel plates of diameter of 25 mm were used, and temperature was controlled by peltier heating.

Figure 6 shows the viscosity of these samples. One can see from the overlap rheology profiles that the viscosity of sample 7 is higher than that of sample K in the shear rate range tested. The blend of Aromox APA-TW and polymer R7-33-158 (a polymer with an amide linkage between the pendant hydrophobe and the backbone) has higher viscosity than Aromox APA-TW alone.

**Comparative Examples L and M - Rheology of Aromox APA-TW and polymer R7-33-28**

Samples L and M was prepared according to the amounts indicated in Table 9.

**Table 9 - Preparation of samples L and M**

| Ex # | Description | Wt. of Polymer R7-33-28, (g) | Wt. of Aromox APA-TW (g) | Wt. of 4% KCl (g) | pH |
|---|---|---|---|---|---|
| L | 3wt% (active) Aromox APA-TW, no polymer, in 4% KCl | 0.0000 | 2.1270 | 27.9093 | 9.72 |

(continued)

| Ex # | Description | Wt. of Polymer R7-33-28, (g) | Wt. of Aromox APA-TW (g) | Wt. of 4% KCl (g) | pH |
|---|---|---|---|---|---|
| M | 3wt% (active) APA-TW + 0.2wt% (active) R7-33-28, in 4% KCl | 0.2335 | 2.1150 | 27.6778 | 8.71 |

For samples L and M, a strain-controlled rheometer ARES (from Rheometric Scientific, which is now TA Instruments) was used to conduct the Steady Strain Rate Sweep at 25°C, with an initial strain rate of 0.01 $s^{-1}$ and a final strain rate of 100 $s^{-1}$. Data was collected, 10 data points per strain rate decade. Parallel plates of diameter of 25 mm were used, and temperature was controlled by peltier heating.

Figure 7 shows the viscosity of these samples. One can see from the overlap rheology profiles that the viscosity of sample M is lower than that of sample L. The blend of Aromox APA-TW and polymer R7-33-28 (a polymer with an ester linkage between the pendant hydrophobe and the backbone) has lower viscosity than Aromox APA-TW alone. In this case, blending VES and polymer showed a "negative" rheology synergy.

**Example 8 and comparative example N - Viscoelasticity of Aromox APA-TW and polymer R7-33-43**

Samples 8 and N was prepared according to the amounts indicated in the following Table 10.

**Table 10 - preparation of samples 8 and N**

| Ex # | Description | Wt. of Polymer (g) | Wt. of Aromox APA-TW (g) | Wt. of 4% KCl (g) | pH |
|---|---|---|---|---|---|
| N | 3wt% (active) Aromox APA-TW, no polymer, in 4% KCl | 0.0000 | 2.1100 | 27.9126 | 10.17 |
| 8 | 3wt% (active) Aromox APA-TW + 0.2wt% (active) R7-33-43, in 4% KCl | 0.4585 | 2.0974 | 27.4362 | 9.26 |

Dynamic Frequency Sweep was tested using a stress-controlled rheometer SR-5000 (originally made by Rheometrics) at 25°C, with initial frequency of 0.01 rad/s and final frequency of 100 rad/s, and stress = 1 Pa. Parallel plates of diameter of 40 mm were used, and temperature was controlled by peltier heating. 10 data points were collected within each decade of frequency. The results are shown in Figures 8 and 9

From Figure 8, one can see the significant difference of the G' and G" profile between the two samples. Sample N had no polymer, and its G', G" crossover frequency was roughly 3 rad/s. The sample showed viscoelasticity, or G' > G", only at frequencies that are higher than 3 rad/s. Sample 8 was the blend of Aromox APA-TW and polymer R7-33-43, and its G', G" crossover frequency was roughly 0.02 rad/s. The sample showed viscoelasticity, or G' > G", at frequencies that are higher than 0.02 rad/s. Therefore, the VES-polymer blend showed much wider frequency range where viscoelastcity was demonstrated.

From Figure 9, one can see the significant difference in the phase angle between these two samples. Sample N had no polymer, and its phase angle is mostly higher than 45 degrees. Its phase angle was only below 45 degrees at frequency higher than 3 rad/s, indicating that it only has viscoelastic characteristics at frequencies higher than 3 rad/s. Sample 8 was the blend of Aromox APA-TW and polymer R7-33-43, and its phase angle was below 45 degrees at frequencies higher than 0.02 rad/s. This is another data to support that the VES-polymer blend is more viscoelastic than VES alone.

**Example 9 and comparative example O - Viscoelasticity of Aromox APA-TW and polymer J3-9-46.**

Samples 9 and O were prepared according to the amounts indicated in the following Table 11.

**Table 11 - Preparation of samples 9 and O**

| Ex. | Description | Wt. of Polymer (g) | Wt. of Aromox APA-TW (g) | Wt. of 4% KCl (g) | pH Measured |
|---|---|---|---|---|---|
| O | 3wt% (active) Aromox APA-TW, no polymer, in 4% KCl | 0.0000 | 2.1270 | 27.9093 | 9.72 |
| 9 | 3wt% (active) Aromox APA-TW + 0.2% (active) J3-9-46, in 4% KCl | 0.4505 | 3.5028 | 46.0249 | 7.75 |

For sample O, a strain-controlled rheometer ARES (from Rheometric Scientific, which is now TA Instruments) was used to conduct the Steady Strain Rate Sweep at 25°C, with an initial strain rate of 0.01 $s^{-1}$ and a final strain rate of 100 $s^{-1}$. Data was collected, 10 data points per strain rate decade. Parallel plates of diameter of 25 mm were used, and temperature was controlled by peltier heating.

Figure 10 shows the results from these experiments. For sample O, a strain-controlled rheometer ARES (from Rheometrics, which is now TA Instrument) was used to conduct the Steady Strain Rate Sweep at 25°C, with an initial strain rate of 0.01 $s^{-1}$ and a final strain rate of 100 $s^{-1}$. Data was collected, 10 data points per strain rate decade. Parallel plates of diameter of 25 mm were used, and temperature was controlled by peltier heating. For sample 9, a stress-controlled rheometer SR-5000 (from Rheometrics, which is now TA Instrument) was used to conduct the Steady Stress Sweep at 25°C, with an initial stress of 0.1 Pa, a final stress of 40 Pa, and a linear stress increment of 0.5 Pa. Parallel plates of diameter of 40 mm were used, and temperature was controlled by peltier heating.

One can see from the overlap rheology profiles that the viscosity of sample 9 is higher than that of sample O in the shear rate range tested. The blend of Aromox APA-TW and polymer J3-9-46 (a cationic hydrophobically modified polymer) has higher viscosity than Aromox APA-TW alone.

**Example 10: Anionic/Betaine Surfactant-Polymer Dilution Experiments**

Sodium lauryl ether sulfate and cocamidopropyl betaine were chosen as exemplary surfactants. They were used in a constant ratio to each other of 4:1 respectively. A series of eight dilution experiments were conducted. The compositions of the two series of experiments are found in Table 12 shown below. Sodium Chloride was the salt used in all the samples in Example 10. The first four dilution experiments (labeled 1-4) represent "no polymer controls". The latter four dilution experiments (labeled 5-8) contain different levels of a polymer R7-36-72 of the current invention. Columns A-D represent fixed levels of surfactant (column A for 15% surfactant, B for 10% surfactant, C for 7.5% surfactant, and D for 6% surfactant). In Table 13, the zero shear viscosities are shown to correlate with the dilution experiments described in Table 12.

**Table 12**

| | A Mass % Initial Sample | B Mass % First Dilution | C Mass % Second Dilution | D Mass % Third Dilution |
|---|---|---|---|---|
| 1 | | | | |
| %Surfactant | 15 | 10 | 7.5 | 6 |
| % Salt | 8 | 5.33 | 4 | 3.2 |
| % Water | 77 | 84.67 | 88.5 | 90.8 |
| 2 | | | | |
| %Surfactant | 15 | 10 | 7.5 | 6 |
| % Salt | 6 | 4 | 3 | 2.4 |
| % Water | 79 | 86 | 89.5 | 91.6 |
| 3 | | | | |
| %Surfactant | 15 | 10 | 7.5 | 6 |
| % Salt | 3 | 2 | 1.5 | 1.2 |
| % Water | 82 | 88 | 91 | 92.8 |
| 4 | | | | |
| %Surfactant | 15 | 10 | 7.5 | 6 |
| % Salt | 2 | 1.33 | 1 | 0.8 |
| % Water | 83 | 88.67 | 91.5 | 93.2 |
| 5 | | | | |
| %Surfactant | 15 | 10 | 7.5 | 6 |
| % Salt | 8 | 5.33 | 4 | 3.2 |
| % Polymer | 0.4 | 0.267 | 0.2 | 0.159 |
| % Water | 76.6 | 84.403 | 88.3 | 90.641 |
| 6 | | | | |
| %Surfactant | 15 | 10 | 7.5 | 6 |
| % Salt | 6 | 4 | 3 | 2.4 |
| % Polymer | 0.4 | 0.267 | 0.2 | 0.159 |
| % Water | 78.6 | 85.733 | 89.3 | 91.44 |
| 7 | | | | |
| %Surfactant | 15 | 10 | 7.5 | 6 |
| % Salt | 3 | 2 | 1.5 | 1.2 |
| % Polymer | 0.4 | 0.267 | 0.2 | 0.159 |
| % Water | 80.6 | 87.06 | 90.3 | 92.241 |
| 8 | | | | |
| %Surfactant | 15 | 10 | 7.5 | 6 |
| % Salt | 2 | 1.33 | 1 | 0.8 |
| % Polymer | 0.4 | 0.267 | 0.2 | 0.159 |
| % Water | 82.6 | 88.403 | 91.3 | 93.041 |

**Table 13**

| *Zero Shear Viscosity (Pa-s) - Surfactant + Polymer R7-36-72* | | | | |
|---|---|---|---|---|
| ***Experiment #*** | ***A*** | ***B*** | ***C*** | ***D*** |
| *1* | *0.004* | *54* | *115* | *16* |
| *2* | *6* | *173* | *31* | *0.4* |
| *3* | *130* | *157* | *0.7* | *0.007* |

(continued)

| *Zero Shear Viscosity (Pa-s) - Surfactant + Polymer R7-36-72* | | | | |
|---|---|---|---|---|
| ***Experiment #*** | ***A*** | ***B*** | ***C*** | ***D*** |
| *4* | *0.001* | *0.001* | *0.001* | *0.001* |
| *5* | *0.7* | *22* | *170* | *58* |
| *6* | *1.3* | *188* | *138* | *1.7* |
| *7* | *31* | *492* | *5.4* | *0.001* |
| *8* | *209* | *17* | *0.001* | *0.001* |

Comparisons to determine influence of polymer at different salt levels can be made between the dilution experiment pairs of 1 and 5, 2 and 6, 3 and 7, and 4 and 8.

Zero Shear Viscosity

Zero shear viscosities were obtained from either a plot of viscosity vs. shear rate or fitting the data to Cross model. Viscosities were measured using a stress-controlled rheometer SR-5000 (from Rheometric Scientific, which is now TA Instruments)a stress controlled rheometer utilizing a 25 mm diameter parallel plate configuration and 1 mm gap. Steady state stress sweep measurements were carried out at 25 $^0$C a data at different shear rate were obtained.

The zero shear viscosity was obtained by averaging the values at the viscosity plateau at the lower end of the shear rate range. For cases when the value does not reach plateau, the data was fitted to the Cross model. The fitting was carried out using the solver module of the MS Excel and setting the criterion to minimize the sum of the percentage differences between the observed and fitted data. According to the Cross model viscosity is related to shear rate by the following equation:

$$\eta = \eta_\infty + \frac{\eta_0 - \eta_\infty}{1 + (C\dot{\gamma})^m}$$

$n_0$ = zero shear viscosity, $\eta_\infty$ = viscosity at very high rate, $\acute{y}$ = □shear rate, *C* and *m* are constants.

| Calculated variables of Cross model | |
|---|---|
| $\eta_0$ | 0.41 Pa-s |
| $\eta_\infty$ | 0.0404 Pa-s |
| *C* | 0.01 |
| *m* | 0.97 |

The plot in Figure 11 displays the results of zero shear viscosity vs. % salt for the 8 dilution experiments from example 10. In this case, experiments #5-8 relate to experiments employing R7-36-72 as the polymer of the present invention.

These data demonstrate that R7-36-72 can effectively increase the zero shear viscosity for the sodium lauryl ether sulfate/cocamidopropyl betaine mixture over all surfactant levels to levels higher than can be achieved without polymer. Additionally, it can generally be seen that the peak viscosity of the system can be achieved with lower salt levels than in the absence of the polymer of the present invention. Furthermore, it can be observed that the zero shear viscosity of a high surfactant containing system can be achieved at substantially lower surfactant content by the addition of the polymer of the present invention with the appropriate level of salt.

The peak viscosity achieved for each surfactant level is provided in Table 14 below. Peak viscosity refers to the highest viscosity achieved over the entire salt range that was tested.

**Table 14**

| R7-36-72 Testing in Surfactant System | Peak Zero Shear Viscosity (Pa-s) |
|---|---|
| 6% surfactant without polymer | 16 |

(continued)

| R7-36-72 Testing in Surfactant System | Peak Zero Shear Viscosity (Pa-s) |
|---|---|
| 6% surfactant with polymer | 58 |
| 7.5% surfactant without polymer | 115 |
| 7.5% surfactant with polymer | 170 |
| 10% surfactant without polymer | 173 |
| 10% surfactant with polymer | 429 |
| 15% surfactant without polymer | 130 |
| 15% surfactant with polymer | 209 |

Comparing samples with and without polymer shows that in all cases, the addition of polymer enables higher zero shear viscosities. Additionally, comparing 15% surfactant without polymer (130 Pa-s) to 10% surfactant without polymer (173 Pa-s) and 10% surfactant with polymer (429 Pa-s) shows that it is possible to achieve higher zero shear viscosities with lower levels of surfactant by addition of the polymer of the present invention and the appropriate level of salt.

**Example 10: Stress Sweep and Elastic Modulus Measurement**

Stress Sweep

Dynamic stress sweep were also evaluated using the Rheometric Scientific rheometer and same plate configuration as for steady state stress sweep. Measurement was carried out at a frequency of 1 Hz. These values allow us to investigate yield stress, storage (elastic) and loss (viscous) modulus. Figure below shows a typical example.

Elastic Modulus Results

Composition 1 containing 7.5% of surfactant, 3% Sodium Chloride, and 0% polymer was prepared and evaluated according to Example 10. Composition 2 containing 7.5% surfactant, 3% Sodium Chloride, and 0.2% R7-36-72 was prepared and evaluated according to Example 5.

The results are found in Figure 9 showing Elastic Modulus as a function of Stress.

These data demonstrate that addition of the polymer of the present invention can dramatically increase the elastic modulus and yield stress of surfactant systems.

**Example 12: Foam Evaluation**

Foam property was evaluated by creating a foam using a blender and the measuring the time taken for half of total liquid to drain from the foam matrix.

**Procedure:**

Prepare a 0.035% solution of each of the formulation by diluting with water. A total of 200 ml of the 0.035% solution were transferred to a blender (Oster Fusion, 3 speed blender). Blend the solution at medium speed for 30 sec. Transfer the resulting foam quickly into a 1000ml cylinder and measure the volume of foam created.

**Example 13: Influence of Polymer on Foam Volume**

Shampoo 1 containing 7.5% surfactant, 3% salt was prepared and evaluated for its foam height. Second, shampoo 2 containing 7.5% surfactant, 3% salt, and 0.25% polymer R7-36-72 was prepared and evaluated for its foam height. Finally, a Shampoo 3, a deep cleansing shampoo from a leading brand that is estimated to contain 15% total surfactants, was evaluated for its foam height. The results are summarized in Table 10 below.

Table 10

| Product Name | Foam Height | Standard Deviation |
|---|---|---|
| Shampoo 1 | 782mL | 30mL |
| Shampoo 2 | 845mL | 2mL |
| Shampoo 3 | 802mL | 3mL |

These data demonstrate that the polymers of the present invention enhance the foam characteristics of shampoo. Additionally, these data demonstrate that it is possible to exceed the foam characteristics of a shampoo with ~15% surfactant with one that contains only 7.5% surfactant by addition of the polymer of the present invention.

All documents cited in the Detailed Description of the Invention are, in relevant part, incorporated herein by reference; the citation of any document is not to be construed as an admission that it is prior art with respect to the present invention.

While particular embodiments of the present invention have been illustrated and described herein, the invention is not intended to be limited to the details shown. Rather, various modifications may be made in the details within the range and scope of equivalents of the claims and without departing from the spirit and scope of the invention.

## Claims

1. An aqueous viscoelastic personal care composition comprising:

   at least one viscoelastic surfactant;
   at least one hydrophobically-modified polymer obtainable by copolymerizing at least a first and at least a second ethylenically unsaturated monomer, wherein

   - said first monomer is an ethylenically unsaturated monomer with a pendant hydrocarbyl group having from 6 to 40 carbon atoms, being connected to the unsaturated function of said monomer via a non-ester containing linkage, preferably a urea, urethane, imide and amide containing linkage, more preferably a urea or urethane containing linkage;
   - said second monomer is an ethylenically unsaturated monomer free from hydrocarbyl groups having 6 or more carbon atoms connected to the unsaturated function of said monomer; and

   a personal care active ingredient.

2. The personal care composition of claim 1 wherein the at least one hydrophobically-modified polymer has a number average molecular weight of from 1000 to 100,000 Da.

3. The personal care composition of claim 1 or 2 wherein the at least one hydrophobically modified polymer, to a level of least 0.1 mol%, based on the amount of monomer units in the polymer, of covalently bonded pendant hydrocarbyl groups having from 6 to 40 carbon atoms.

4. The personal care composition of any one of claims 1-3, wherein said pendant hydrocarbyl group has at least 12 carbon atoms and 0.1 to 20 mole%, preferably from 0.5 to 10 mole%, more preferably from 1 to 5 mole%, based on the amount of monomer units in the polymer, of the monomeric units of the polymer are connected to said pendant hydrocarbyl groups.

5. The personal care composition of any one of claims 1-3, wherein said pendant hydrocarbyl group has at most 11 carbon atoms and from 1 to 50 mole%, preferably from 5 to 40 mole%, more preferably from 10 to 30 mole%, based on the amount of monomer units in the polymer, are connected to said pendant hydrocarbyl group.

6. The personal care composition of any one of claims 1-3, wherein said pendant hydrocarbyl group contains at least 8, preferably at least 11 carbon atoms.

7. The personal care composition according to any one of the preceding claims, wherein said pendant hydrocarbyl group contains at most 32, preferably at most 24 carbon atoms.

**8.** The personal care composition according to any one of the preceding claims, wherein said pendant hydrocarbyl group is a branched alkyl or alkenyl group.

**9.** The personal care composition according to any one of the preceding claims, wherein said hydrophobically modified polymer is present in a concentration below the overlap concentration of said polymer.

**10.** The personal care composition according to any one of the preceding claims, wherein said first and second monomer are present in a mutual molar ratio of from 0.1:99.9 to 90:10

**11.** The personal care composition according to claim 10, wherein said hydrocarbyl group has at least 12 carbon atoms and said first and second monomers are present in a molar ratio of from 0.1:99.9 to 20:80; preferably from 0.5:99.5 to 10:90, more preferably from 1:99 to 5:95, or wherein said hydrocarbyl group has at most 11 carbon atoms and said first and second monomers are present in a mutual molar ratio of from 1:99 to 90:10; preferably from 5:95 to 70:30, more preferably from 10:90 to 50:50.

**12.** The personal care composition according to any one of claims 10 to 12, wherein said second ethylenically unsaturated monomer is selected from the group consisting of anionic ethylenically unsaturated monomers, cationic ethylenically unsaturated monomers, non-ionically ethylenically unsaturated monomers, zwitterionic ethylenically unsaturated monomers, and mixtures thereof and salts thereof.

**13.** The personal care composition according to any one of the preceding claims, wherein said viscoelastic surfactant is selected from the group consisting of zwitterionic surfactants, amphoteric surfactants, anionic surfactants and combinations thereof.

**14.** The personal care composition according to any one of the preceding claims, wherein the weight ratio of hydrophobically modified polymer to viscoelastic surfactant is from 0.1:100, preferably from 1:100, more preferably from 3:100, to 100:50, preferably to 100:100, more preferably to 50:100.

**15.** Use of the personal care composition of any one of the preceding claims as a liquid cleansing product for hair or skin, a liquid conditioning product for hair or skin, or a shaving preparation.

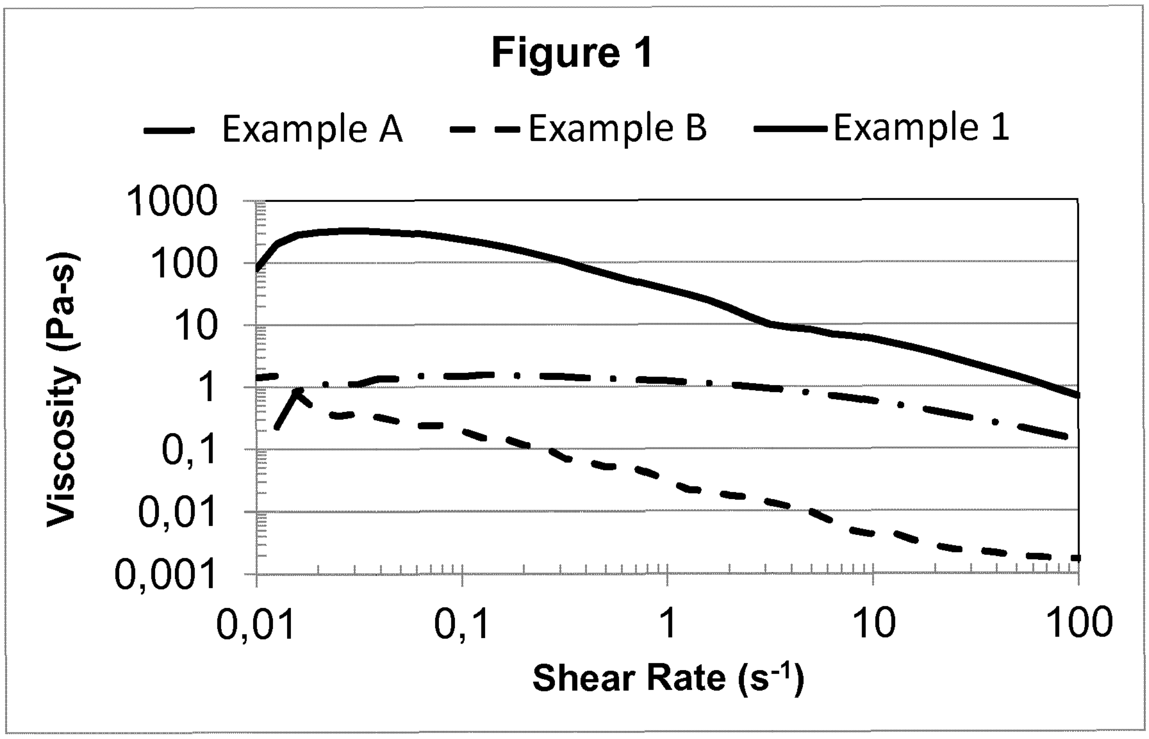
Figure 1
Example A
Example B
Example 1
Viscosity (Pa-s)
1000
100
10
1
0,1
0,01
0,001
0,01
0,1
1
10
100
Shear Rate (s-1)

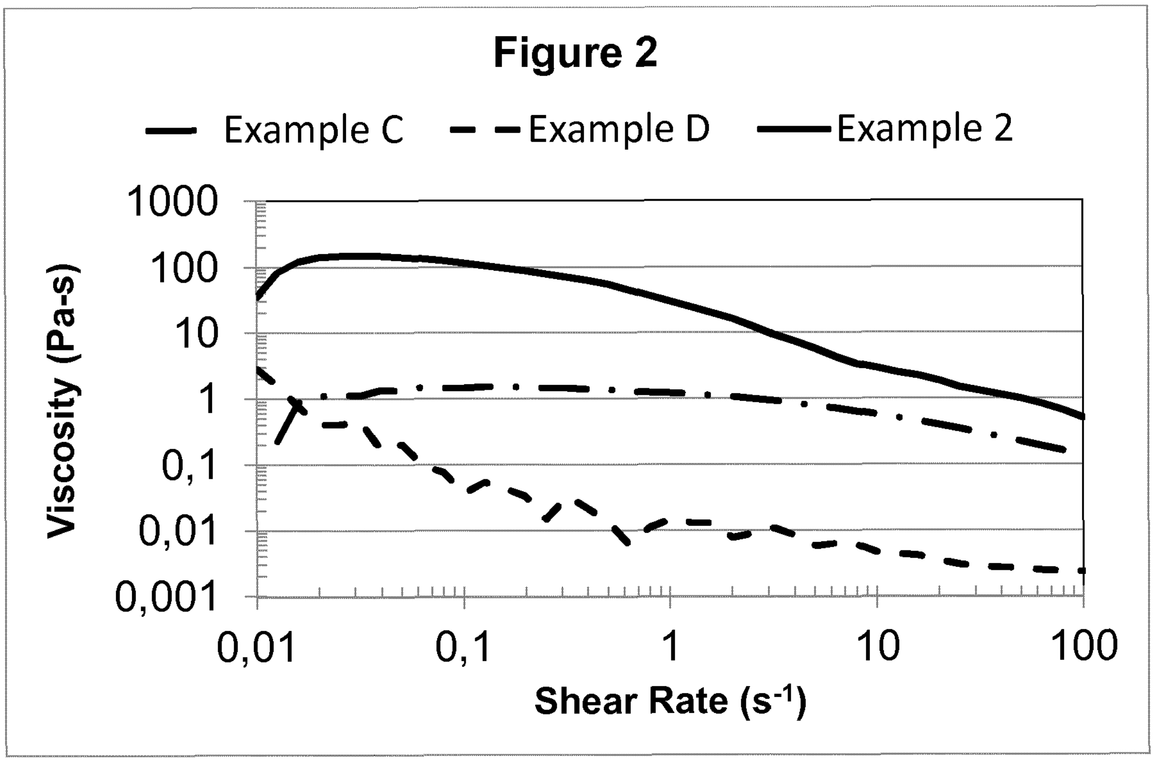
Figure 2
Example C
Example D
Example 2
Viscosity (Pa-s)
1000
100
10
1
0,1
0,01
0,001
0,01
0,1
1
10
100
Shear Rate (s-1)

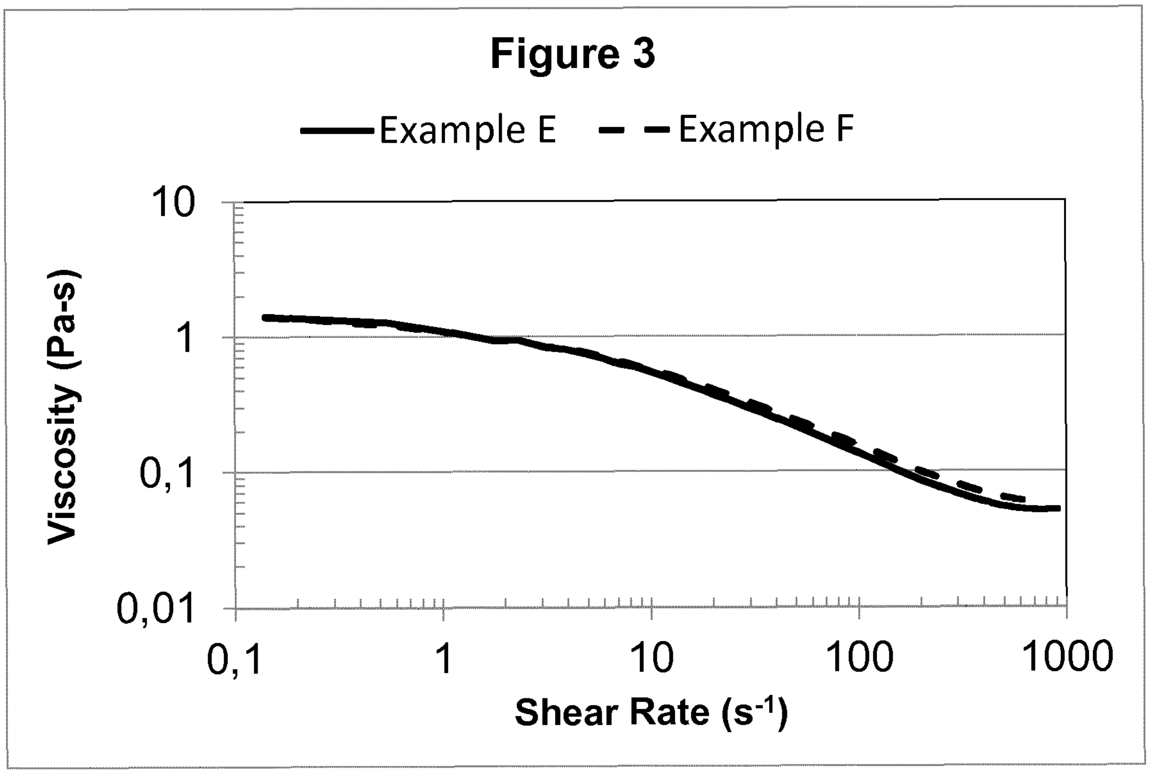
Figure 3
Example E
Example F
Viscosity (Pa-s)
10
1
0,1
0,01
0,1
1
10
100
1000
Shear Rate (s-1)

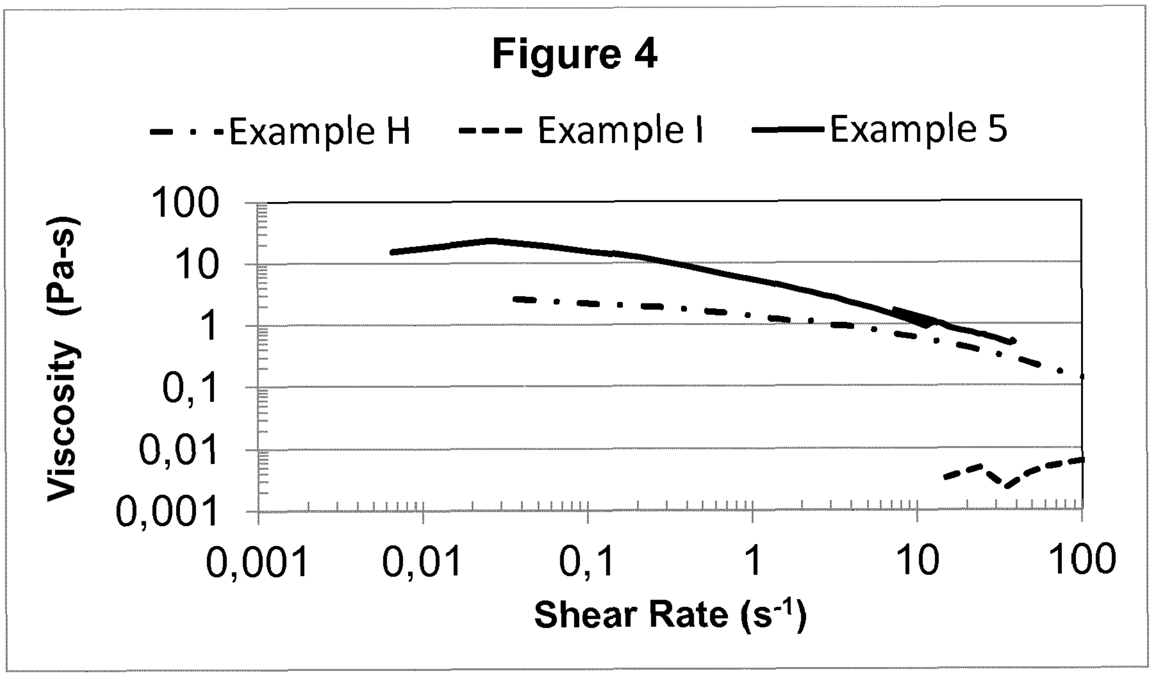
Figure 4
Example H
Example I
Example 5
Viscosity (Pa-s)
100
10
1
0,1
0,01
0,001
0,001
0,01
0,1
1
10
100
Shear Rate (s-1)

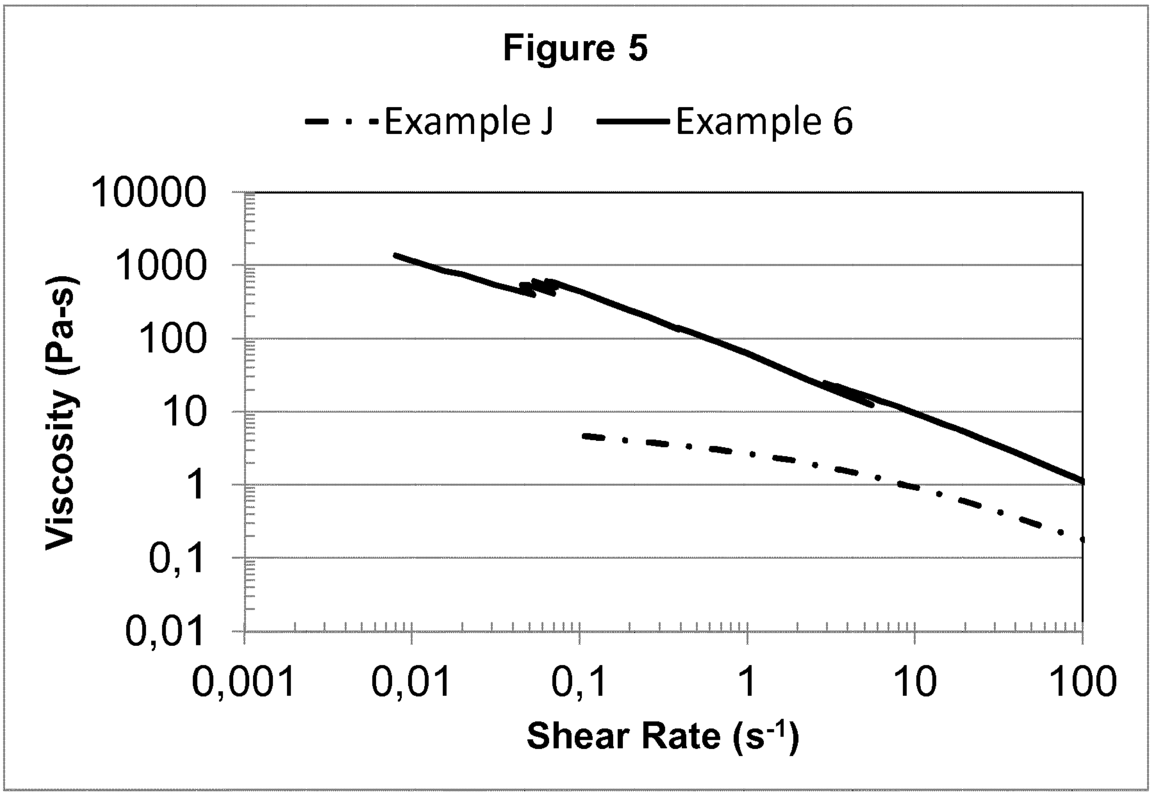
Figure 5
Example J
Example 6
Viscosity (Pa-s)
10000
1000
100
10
1
0,1
0,01
0,001
0,01
0,1
1
10
100
Shear Rate (s-1)

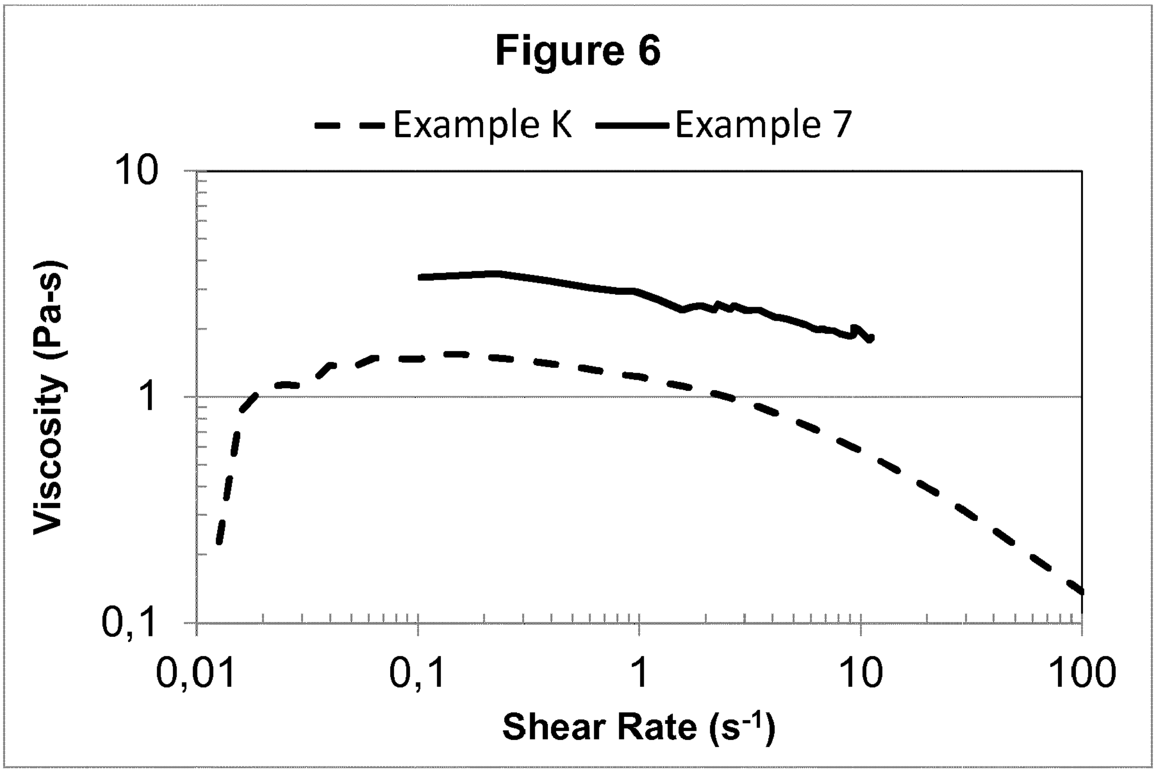
Figure 6
Example K
Example 7
Viscosity (Pa-s)
10
1
0,1
0,01
0,1
1
10
100
Shear Rate (s-1)

Figure 7

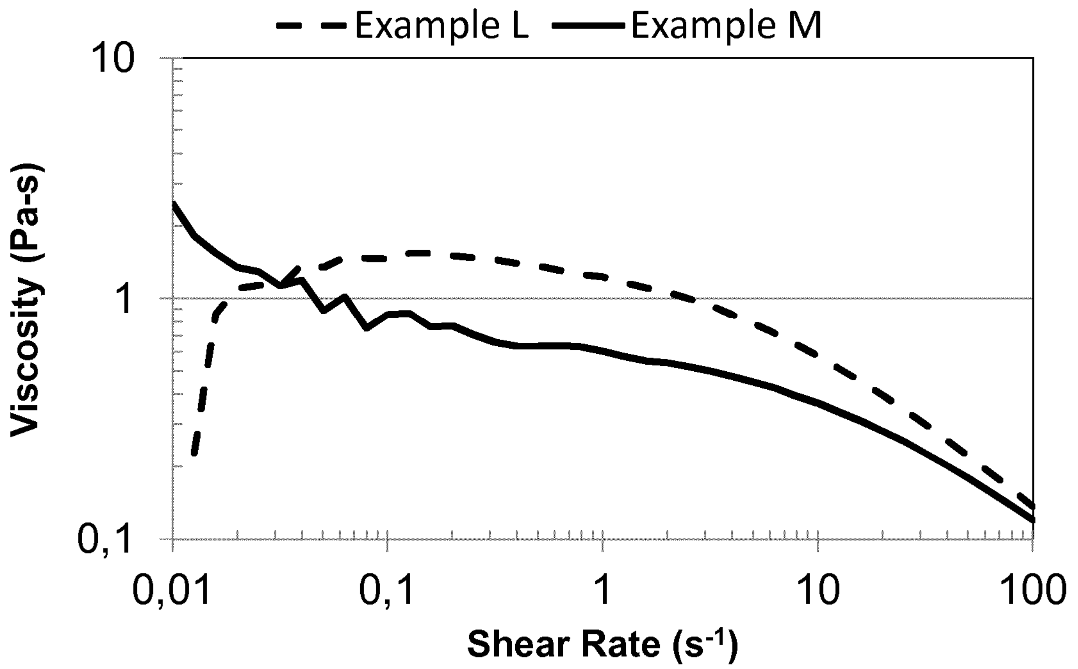


Figure 8

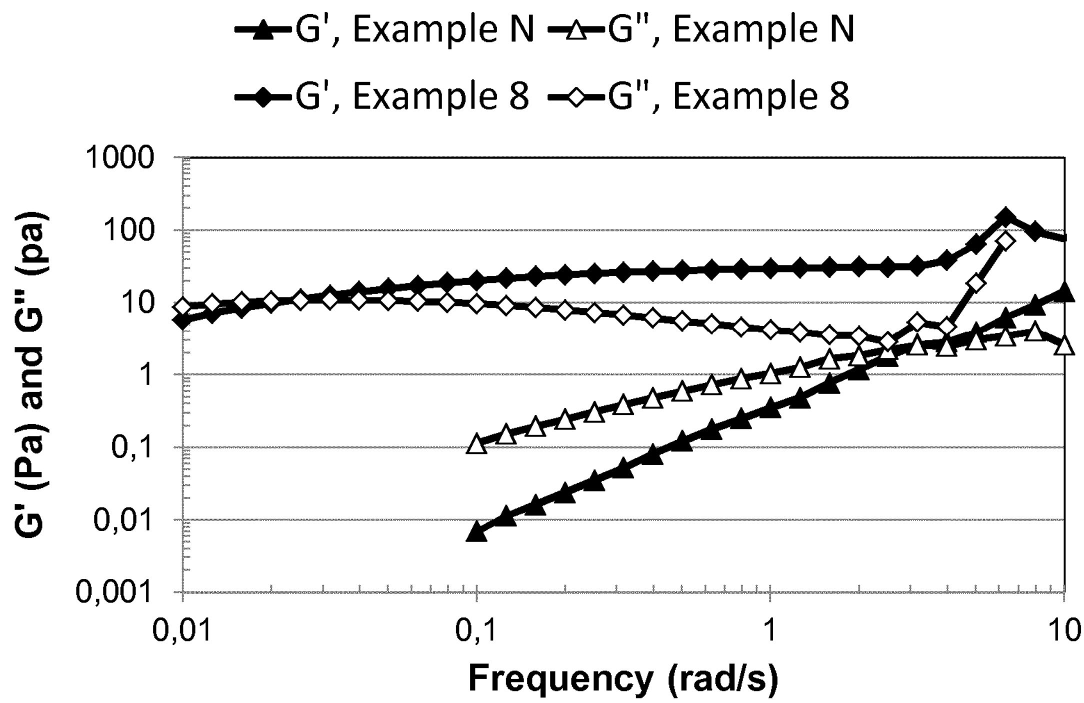

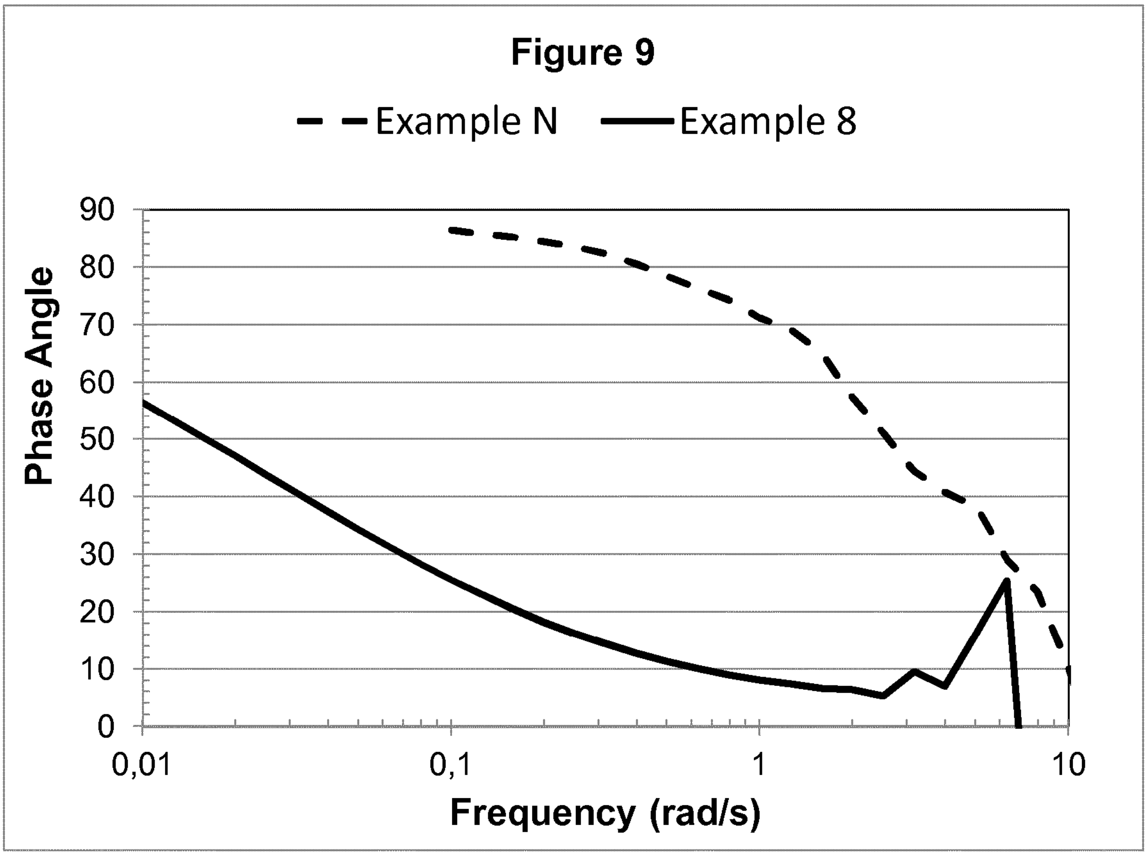
Figure 9
Example N
Example 8
Phase Angle
90
80
70
60
50
40
30
20
10
0
0,01
0,1
1
10
Frequency (rad/s)

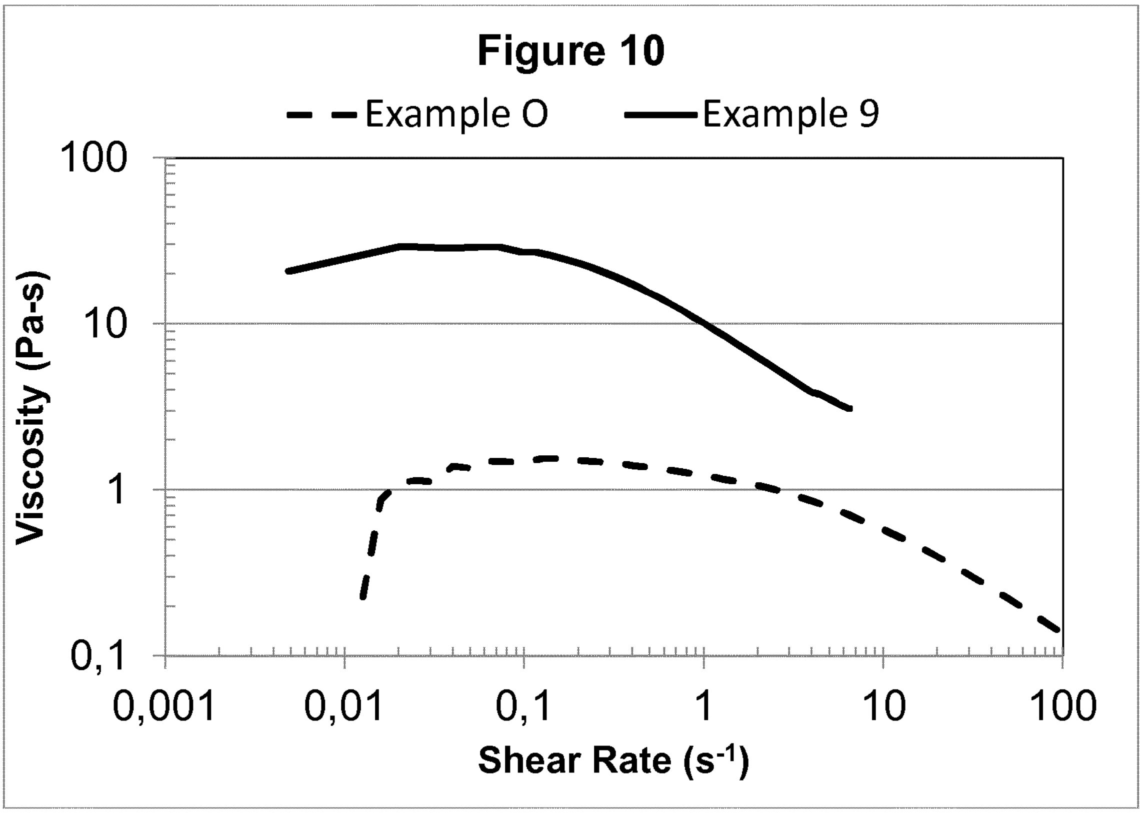
Figure 10
Example O
Example 9
Viscosity (Pa-s)
100
10
1
0,1
0,001
0,01
0,1
1
10
100
Shear Rate (s-1)

Figure 11 – Zero Shear Viscosity

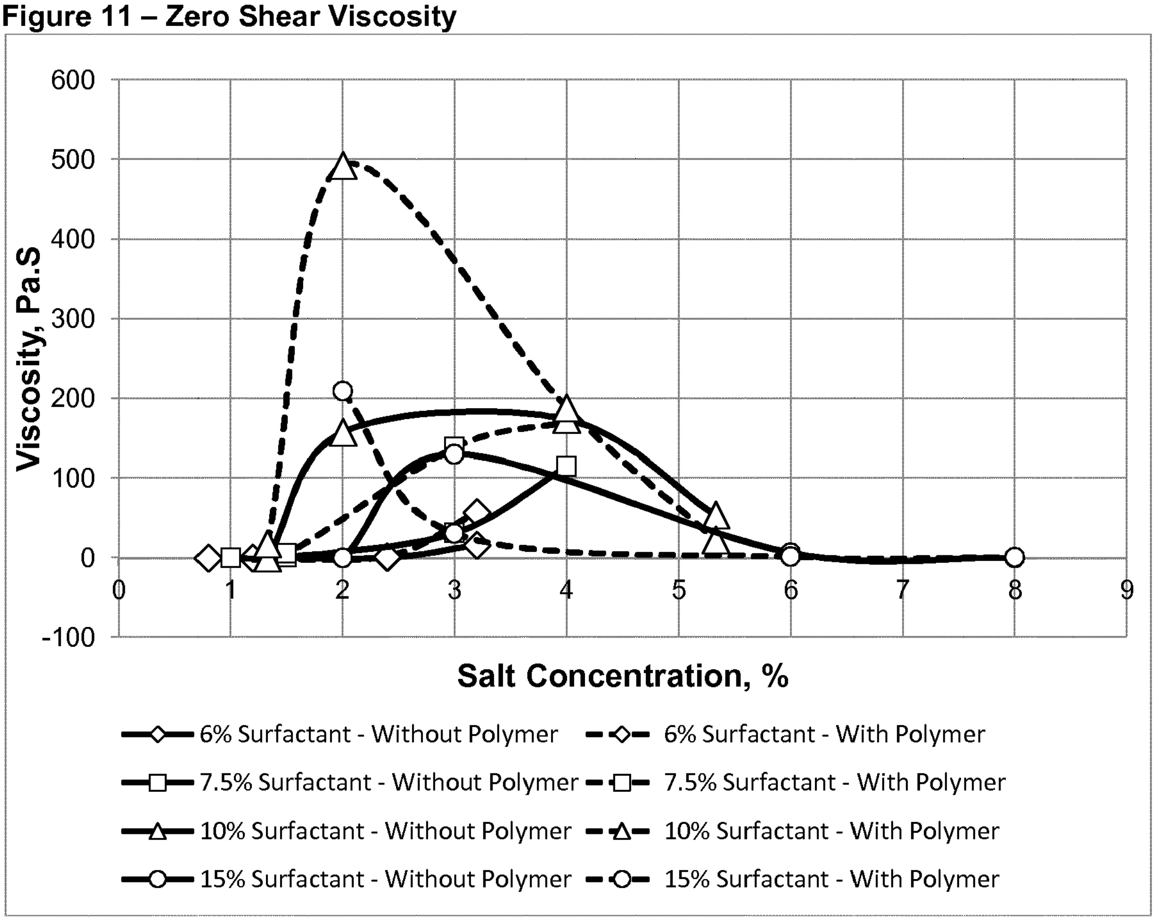

Europäisches Patentamt
European Patent Office
Office européen des brevets

# EUROPEAN SEARCH REPORT

Application Number
EP 15 16 8141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/036160 A1 (NALCO CO [US]; HESSEFORT YIN Z [US]; MAREK PATRICK J [US]; CRAMM JEFFR) 19 March 2009 (2009-03-19) * claims 1,14,15,16,18 * ----- | 1-15 | INV. C09K8/60 C09K8/88 A01N25/00 C11D3/37 A01N25/10 A61Q19/00 |
| Y | WO 2005/023970 A1 (JOHNSON & JOHNSON CONSUMER [US]; LIBRIZZI JOSEPH [US]; MARTIN ALISON [) 17 March 2005 (2005-03-17) * claims 1-38 * ----- | 1-15 | |
| Y | US 2006/142501 A1 (BADIGER MANOHAR V [IN] ET AL) 29 June 2006 (2006-06-29) * claims 1-14 * ----- | 1-15 | |
| A | US 2007/111896 A1 (KNOX PAUL W [US] ET AL) 17 May 2007 (2007-05-17) * claims 1,6,26,35 * ----- | 1-15 | |

TECHNICAL FIELDS SEARCHED (IPC)

C09K
E21B
A01N
C11D
A61Q

The present search report has been drawn up for all claims

1

| Place of search | Date of completion of the search | Examiner |
|---|---|---|
| Munich | 9 September 2015 | Wörth, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 16 8141

This annex lists the patent family members relating to the patent documents cited in the above-mentioned European search report. The members are as contained in the European Patent Office EDP file on
The European Patent Office is in no way liable for these particulars which are merely given for the purpose of information.

09-09-2015

| Patent document cited in search report | Publication date | Patent family member(s) | Publication date |
|---|---|---|---|
| WO 2009036160 A1 | 19-03-2009 | AU 2008298942 A1 | 19-03-2009 |
| | | BR PI0815459 A2 | 18-02-2015 |
| | | CA 2698887 A1 | 19-03-2009 |
| | | CN 101802084 A | 11-08-2010 |
| | | DK 2188336 T3 | 02-12-2013 |
| | | EP 2188336 A1 | 26-05-2010 |
| | | ES 2443120 T3 | 17-02-2014 |
| | | JP 2010539100 A | 16-12-2010 |
| | | KR 20100075893 A | 05-07-2010 |
| | | PT 2188336 E | 11-11-2013 |
| | | SG 185253 A1 | 29-11-2012 |
| | | US 2009069522 A1 | 12-03-2009 |
| | | US 2010093582 A1 | 15-04-2010 |
| | | WO 2009036160 A1 | 19-03-2009 |
| WO 2005023970 A1 | 17-03-2005 | AU 2004270663 A1 | 17-03-2005 |
| | | AU 2004270669 A1 | 17-03-2005 |
| | | AU 2011201479 A1 | 21-04-2011 |
| | | BR PI0413925 A | 24-10-2006 |
| | | BR PI0413992 A | 14-11-2006 |
| | | CA 2537133 A1 | 17-03-2005 |
| | | CA 2539473 A1 | 17-03-2005 |
| | | EP 1660620 A1 | 31-05-2006 |
| | | EP 1673424 A1 | 28-06-2006 |
| | | EP 2343043 A2 | 13-07-2011 |
| | | ES 2528668 T3 | 11-02-2015 |
| | | JP 2007504137 A | 01-03-2007 |
| | | JP 2007504140 A | 01-03-2007 |
| | | KR 20070041416 A | 18-04-2007 |
| | | KR 20070094695 A | 21-09-2007 |
| | | WO 2005023969 A1 | 17-03-2005 |
| | | WO 2005023970 A1 | 17-03-2005 |
| US 2006142501 A1 | 29-06-2006 | NONE | |
| US 2007111896 A1 | 17-05-2007 | CA 2629744 A1 | 24-05-2007 |
| | | EP 1951840 A1 | 06-08-2008 |
| | | EP 2256175 A2 | 01-12-2010 |
| | | ES 2430994 T3 | 22-11-2013 |
| | | US 2007111896 A1 | 17-05-2007 |
| | | WO 2007059266 A1 | 24-05-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003056130 A
- US 4432881 A
- US 2486921 A
- US 2486922 A
- US 2396278 A
- US 3332880 A
- US 3929678 A
- US 2528378 A


### Non-patent literature cited in the description


- **HOFFMANN et al.** Influence of Ionic Surfactants on the Viscoelastic Properties of Zwitterionic Surfactant Solutions. *Langmuir,* 1992, vol. 8, 2140-2146
- **BARNES H. A. et al.** An Introduction to Rheology. Elsevier, 1997, 45-54
- McCutcheon's Emulsifiers and Detergents. MC Publishing, 1989
- **PIERRE-GILLES DE GENNES.** Scaling Concept on Polymer Physics. Cornell University Press, 1979, 76-77